(19) Europäisches Patentamt
European Patent Office
Office européen des brevets

(11) Veröffentlichungsnummer: **0 533 130 A1**

(12) **EUROPÄISCHE PATENTANMELDUNG**

(21) Anmeldenummer: **92115831.7**

(22) Anmeldetag: **16.09.92**

(51) Int. Cl.5: **C07D 213/81**, C07D 213/82, C07D 213/89, A61K 31/44

(30) Priorität: **19.09.91 DE 4131219**
**05.11.91 DE 4136346**

(43) Veröffentlichungstag der Anmeldung:
**24.03.93 Patentblatt 93/12**

(84) Benannte Vertragsstaaten:
**AT BE CH DE DK ES FR GB GR IE IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Weidmann, Klaus, Dr.**
**Scheibenbuschweg 2**
**W-6242 Kronberg/Ts.(DE)**
Erfinder: **Bickel, Martin, Dr.**
**Mittelstedter Weg 3**
**W-6380 Bad Homburg(DE)**
Erfinder: **Kesseler, Kurt, Dr.**
**Gluckstrasse 20a**
**W-6232 Bad Soden am Taunus(DE)**
Erfinder: **Scharbert, Bernd, Dr.**
**Werner-Bockelmann-Strasse 17**
**W-6230 Frankfurt am Main 71(DE)**

(54) **2-Hydroxymethylpyridine, die entsprechenden Pyridin-N-oxide und ihre Derivate, Verfahren zu ihrer Herstellung sowie deren Verwendung.**

(57) Beschrieben werden Pyridinderivate der allgemeinen Formel (I)

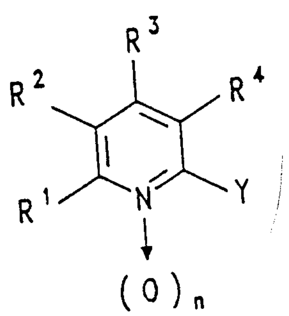

in der Y = Methyl oder $CH_2OR^5$ ,
und ihre Verwendung als Arzneimittel zur Anwendung als Fibrosupressiva und zur Beeinflussung des Stoffwechsels von Kollagen und kollagenähnlichen Stoffen.

EP 0 533 130 A1

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).

In der EP-A-0 176 741 werden Pyridin-2,4- und -2,5-dicarbonsäurediester mit 1-6 C-Atomen im Esteralkylteil als Arzneimittel zur Inhibierung der Prolin- und Lysinhydroxylase beschrieben. Diese niedrigalkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die EP-A-0 278 453, EP-A-0 278 454 und EP-A-0 278 452 beschreiben in allgemeiner Form gemischte Ester/Amide, höher alkylierte Diester und Diamide der Pyridin-2,4- und -2,5-dicarbonsäure, die Kollagenbiosynthese imTiermodell wirksam hemmen.

So wird in der EP-A-0 278 453 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In der europäischen Patentanmeldung EP-A-0 353 668 wird ein verbessertes Verfahren zur Herstellung von N,N'-Bis(2-methoxyethyl)-pyridin-2,4-dicarbonsäurediamid vorgeschlagen. Die europäische Patentanmeldung EP-A-0 409 119 schlägt neue N,N`-Bis(alkoxyalkyl)-pyridin-2,4-dicarbonsäurediamide vor.

In der deutschen Patentanmeldung P 40 01 002.3 werden Pyridin-2,4- und - 2,5-dicarbonsäure-di-(nitroxyalkyl)amide zur Inhibierung der Kollagenbiosynthese vorgeschlagen.

Es bestand die Notwendigkeit nach anderen Verbindungen zu suchen, die eine stärkere antifibrotische Wirksamkeit aufweisen.

Überraschenderweise wurde nun gefunden, daß Pyridinderivate, die in 2-Position eine Methyl- oder eine ggf. mit einer Alkoholschutzgruppe geschützten Hydroxymethylgruppe aufweisen, eine stärkere antifibrotische Wirkung besitzen.

Gegenstand der Erfindung sind daher Pyridinderivate der allgemeinen Formel I

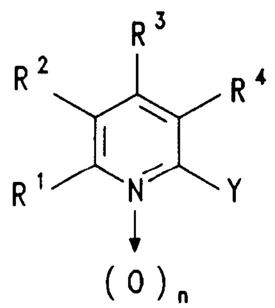

(I)

in der

$R^1$, $R^4$ und einer der beiden Reste $R^2$ oder $R^3$ gleich oder verschieden sind und Wasserstoff, Halogen, insbesondere Fluor, Chlor oder Brom, Cyano, Nitro, Trifluormethyl, $(C_1\text{-}C_{12})$-Alkyl, Hydroxy, $(C_1\text{-}C_6)$-Hydroxyalkyl, $(C_1\text{-}C_{12})$-Alkoxy, $-O-[CH_2-]_xCH_{(2f+1-g)}F_g$, $-OCF_2Cl$, $-O-CF_2-CHFCl$, $(C_1\text{-}C_8)$-Alkylmercapto, $(C_1\text{-}C_8)$-Alkylsulfinyl, $(C_1\text{-}C_8)$-Alkylsulfonyl, $(C_1\text{-}C_8)$-Alkylcarbonyl, $(C_1\text{-}C_6)$-Alkoxycarbonyl, Carbamoyl, N-$(C_1\text{-}C_4)$-Alkylcarbamoyl, N,N-Di-$(C_1\text{-}C_4)$-alkylcarbamoyl, $(C_1\text{-}C_8)$-Alkylcarbonyloxy, $(C_3\text{-}C_8)$-Cycloalkyl, Phenyl, Benzyl, Phenoxy, Benzyloxy, Anilino, N-Methylanilino, Phenylmercapto, Phenylsulfonyl, Phenylsulfinyl, oder
-NR'-R'', $(C_1\text{-}C_{12})$-Alkoxy-$(C_1\text{-}C_{12})$-alkyl, $(C_1\text{-}C_{12})$-Alkoxy-$(C_1\text{-}C_{12})$-alkoxy, bedeuten, oder
einen unsubstituierten oder substituierten $(C_6\text{-}C_{12})$-Aryloxyrest oder
$(C_7\text{-}C_{11})$-Aralkyloxyrest bedeuten, der 1,2,3,4 oder 5 gleiche oder
verschiedene Substituenten aus der Reihe Halogen, Cyano, Nitro, Trifluormethyl, $(C_1\text{-}C_6)$-Alkyl, $(C_1\text{-}C_6)$-Hydroxyalkyl, $(C_1\text{-}C_6)$-Alkoxy, $-O-[CH_2-]_xCH_{2f+1-g)}F_g$, $-OCF_2Cl$, $-O-CF_2-CHFCl$,

$(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, ( $C_1-C_6$)-Alkylcarbonyloxy, $(C_3-C_8)$-Cycloalkyl, Phenyl, Benzyl, Phenoxy, Benzyloxy, NR'-R", Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl insbesondere bis zu 3 der vorstehend genannten gleichen oder verschiedenen Substituenten im Arylteil trägt,
und der andere Rest, $R^2$ oder $R^3$, folgender allgemeiner Formel II entspricht

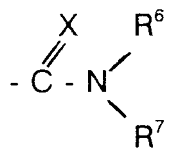
(II)

worin

X O oder S,

$R^6$ einen verzweigten oder unverzweigten $(C_1-C_{12})$-Alkylrest, $(C_1-C_{12})$-Alkenyl- oder $(C_1-C_{12})$-Alkinylrest bedeutet, der einfach oder mehrfach substituiert ist mit
Halogen, Hydroxy, Cyano, Amino, Carboxyl, $(C_1-C_{10})$-Alkoxy, $(C_1-C_{12})$-Alkoxycarbonyl, $(C_3-C_8)$-Cycloalkoxycarbonyl, $(C_7-C_{16})$-Aralkoxycarbonyl, $(C_6-C_{12})$-Aryloxycarbonyl, $(C_1-C_{12})$-Alkylcarbonyloxy, $(C_1-C_{12})$-Alkanoylamino, $(C_3-C_8)$-Cycloalkanoylamino, $C_1-C_{12}$)-Hydroxyalkanoylamino, ( $C_6-C_{12}$)-Aroylamino,$(C_7-C_{11})$-Aralkanoylamino, $(C_1-C_8)$-Alkoxy-$(C_1-C_{12})$-alkanoylamino, $(C_1-C_8)$-Alkoxycarbonyloxy, $(C_1-C_8)$-Alkoxy-$(C_1-C_8)$-alkoxycarbonyloxy, $(C_6-C_{12})$-Aryloxycarbonyloxy, $(C_7-C_{11})$-Aralkyloxycarbonyloxy, $(C_7-C_{11})$-Aralkylcarbonyloxy, $(C_6-C_{12})$-Arylcarbonyloxy, $(C_3-C_8)$-Alkenylcarbonyloxy, $(C_3-C_8)$-Alkinylcarbonyloxy, $(C_3-C_8)$-Cycloalkylcarbonyloxy, $(C_1-C_{12})$-Alkoxy-( $C_1-C_{12}$)-alkoxy, $(C_1-C_{12})$-Alkoxy-amino, $(C_1-C_{12})$-Alkoxy-N-$(C_1-C_6)$-alkylamino, $(C_1-C_{12})$-Alkoxy-N,N-$(C_1-C_6)$-dialkylamino, Carbamoyloxy, N-$(C_1-C_8)$-Alkylcarbamoyloxy, N,N-Di$(C_1-C_8)$-alkylcarbamoyloxy, N-$(C_3-C_8)$-Cycloalkylcarbamoyloxy, $(C_1-C_8)$-Alkylmercapto, $(C_1-C_8)$-Alkylsulfinyl, $(C_1-C_8)$-Alkylsulfonyl, $(C_1-C_8)$-Alkylcarbonyl, $(C_3-C_8)$-Cycloalkylcarbonyl, Nitro, Trifluormethyl, Phenylmercapto, Phenylsulfonyl, Phenylsulfinyl, Sulfamoyl, N-$(C_1-C_6)$-Alkylsulfamoyl, N,N-Di-$(C_1-C_6)$-alkylsulfamoyl, $(C_1-C_8)$-Alkylsulfonamido, Arylsulfonamido, Carbamoyl, N-$(C_1-C_8)$-Alkylcarbamoyl,N,N-Di-$(C_1-C_8)$-alkylcarbamoyl, N-$(C_3-C_8)$-Cycloalkylcarbamoyl, N-$(C_6-C_{12})$-Arylcarbamoyl, N-$(C_7-C_{16})$-Aralkylcarbamoyl, N-$(C_1-C_{10})$-Alkyl-N-$(C_6-C_{12})$-arylcarbamoyl, N-$(C_1-C_{10})$-Alkyl-N-$(C_7-C_{16})$-aralkylcarbamoyl, N-($(C_1-C_8)$-Alkoxy-$(C_1-C_4)$alkyl)carbamoyl, N-($(C_1-C_4)$-Hydroxyalkyl)carbamoyl oder ein O-acyliertes Derivat hiervon, oder mit
einem substituierten $(C_6-C_{12})$-Arylrest, einem $(C_7-C_{11})$Aralkylrest oder einem Heteroarylrest, der 1,2,3,4 oder 5 gleiche oder verschiedene Substituenten aus der Reihe Hydroxy, Halogen, Cyano, Nitro, $(C_1-C_6)$-Alkyl, $(C_1-C_6)$-Alkoxy, Trifluormethyl, $(C_1-C_6)$-Hydroxyalkyl, $-O-[CH_2-]_xC_fH_{(2f+1-g)}F_g$, $-OCF_2Cl$, - $OCF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_8)$-Alkylcarbamoyl, N,N-Di-$(C_1-C_8)$-alkylcarbamoyl, $(C_1-C_6)$-Alkylcarbonyloxy, $(C_3-C_8)$-Cycloalkyl, Phenyl, Benzyl, Phenoxy, Benzyloxy, NR'-R", Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl, insbesondere bis zu 3 der vorstehend genannten gleichen oder verschiedenen Substituenten im Arylteil trägt, oder mit
einem substituierten $(C_6-C_{12})$-Aryloxyrest, $(C_7-C_{11})$Aralkyloxyrest oder einem Heteroaryloxyrest, der 1,2,3,4 oder 5 gleiche oder verschiedene Substituenten aus der Reihe Hydroxy, Halogen, Cyano, Nitro, $(C_1-C_6)$-Alkyl, $(C_1-C_6)$-Alkoxy, Trifluormethyl, $(C_1-C_6)$-Hydroxyalkyl, $-O-[CH_2-]_xC_fH_{(2f+1-g)}F_g$, - $OCF_2Cl$, $-OCF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, NR'-R", Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl, insbesondere bis zu 3 der vorstehend genannten gleichen oder verschiedenen Substituenten im Arylteil trägt,

$R^6$ einen $(C_1-C_{12})$-Alkoxy-, $(C_3-C_8)$-Cycloalkoxyrest oder
einen $(C_6-C_{12})$-Arylrest, $(C_7-C_{11})$-Aralkylrest oder einen Heteroarylrest bedeutet, der
1,2,3,4 oder 5 gleiche oder verschiedene Substituenten aus der Reihe Hydroxy, Halogen, Cyano, Nitro, $(C_1-C_6)$-Alkyl, $(C_1-C_6)$-Alkoxy, Trifluormethyl, $(C_1-C_6)$-Hydroxyalkyl, $-O-[CH_2-]_xC_fH_{(2f+1-g)}F_g$,

-$OCF_2Cl$, -$OCF_2$-CHFCl, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl N-($C_1$-$C_8$)-Alkylcarbamoyl, N,N-Di-($C_1$-$C_8$)-alkylcarbamoyl, N-($C_3$-$C_8$)-Cycloalkylcarbamoyl, N-($C_6$-$C_{12}$)-Arylcarbamoyl, N-($C_7$-$C_{16}$)-Aralkylcarbamoyl, N-($C_1$-$C_{10}$)-Alkyl-N-($C_6$-$C_{12}$)arylcarbamoyl, N-($C_1$-$C_{10}$)-Alkyl-N-($C_7$-$C_{16}$)aralkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R", Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl insbesondere bis zu 3 der vorstehend genannten gleichen oder verschiedenen Substituenten im Arylteil trägt,
worin jeweils
R' und R" gleich oder verschieden sind und Wasserstoff, ($C_6$-$C_{12}$)-Aryl, ($C_1$-$C_8$)-Alkyl, ($C_1$-$C_8$)-Aminoalkyl, N-($C_1$-$C_8$)-Alkylamino-($C_1$-$C_{12}$)-alkyl, N,N-Di-($C_1$-$C_8$)-alkylamino-($C_1$-$C_{12}$)-alkyl, ($C_7$-$C_{14}$)-Aralkyl, bedeuten oder gemeinsam für - $[CH_2]_n$- stehen, worin eine $CH_2$-Gruppe durch O,S,N-($C_1$-$C_4$)-Alkanoylimino oder N-($C_1$-$C_4$)-Alkoxycarbonylamino ersetzt sein kann,

und

$R^6$ substituiert sein kann mit einem Rest der allgemeinen Formel III

- O - Z (III)

worin

Z eine über ihren Acylrest gebundene Aminosäure oder eines Derivates hiervon oder $R^5$ bedeutet, oder mit einem Rest der allgemeinen Formel IV

- CO - U (IV)

worin

U ein über die Aminogruppe gebundenes Polypeptid, vorzugsweise eine Tri-oder ein Dipeptid, oder ein über die Aminogruppe gebundenes Aminosäurederivat, vorzugsweise ein L-Aminosäure-Derivat, insbesondere ein L-Alanin- oder ein Glycinderivat, bedeutet,

$R^7$ unabhängig von $R^6$ Wasserstoff oder $R^6$ sein kann, wobei die Reste $R^6$ und $R^7$ auch zusammen mit dem Stickstoffatom einen 5-, 6- oder 7-gliedrigen gesättigten heterocyclischen Ring bilden, der heterocyclische Ring auch ein zweites Stickstoffatom beinhalten kann und der heterocyclische Ring seinerseits mit Phenyl oder Phenyl-$C_1$-$C_3$-Alkyl substituiert sein kann,

Y Methyl oder $CH_2$-$OR^5$ bedeutet, wobei

$R^5$ Wasserstoff, Z oder den Rest einer physiologisch verträglichen, vorzugsweise in der Leber oder unter physiologischen Bedingungen abspaltbaren Alkohol-Schutzgruppe bedeutet, und

n 0 der 1

f 1 bis 8, vorzugsweise 1 bis 5

g 0,1 bis (2f + 1) sowie

x 0, 1, 2 oder 3, vorzugsweise 0 oder 1 ist,

zuzüglich alle Derivate, die in der entsprechenden Amino- oder Hydroxygruppe eine Schutzgruppe aufweisen sowie die physiologisch wirksamen Salze.

Unter Halogen werden Fluor, Chlor, Brom und Jod, unter Aryl Phenyl und Naphthyl und unter Heteroaryl 5- und 6-gliedrige aromatische Ringe mit 1,2- oder 3 Stickstoff- und/oder Sauerstoff- und/oder Schwefelatomen verstanden, die gegebenenfalls noch benzoanneliert sein können; insbesondere handelt es sich bei den Heteroarylresten um Pyridyl-, Pyridazyl-, Pyrimidyl, Pyrazyl-, 1,3,5-Triazyl-Pyrolyl-, Pyrazolyl-, Imidazolyl-, Triazolyl-, Thienyl-, Oxazolyl- und Thiazolyl-Reste und gegebenenfalls deren benzoannelierte Derivate.

Unter einem Alkylrest ist ein linearer, verzweigter oder cyclischer Rest mit 1-12 Kohlenstoffatomen zu verstehen. Sofern cyclische Alkylreste vorliegen, weisen diese 3-10, vorzugsweise 5 oder 6 Kohlenstoffatome auf. Verzweigte Alkylreste sind umso häufiger bevorzugt, je größer die Anzahl der C-Atome ist.

Die Alkylreste können auch einfach oder mehrfach ungesättigt sein. Bevorzugt sind allerdings gesättigte Alkylreste. Sofern aber ungesättigte Reste vorliegen, handelt es sich bevorzugt um lineare unverzweigte Reste.

Die genannten Alkylreste können sowohl chirale, wie achirale Kohlenstoffatome enthalten.

Unter dem Rest ($C_7$-$C_{11}$)-Aralkyloxy ist vorzugsweise ein substituierter Phenylalkyloxyrest der Formel V

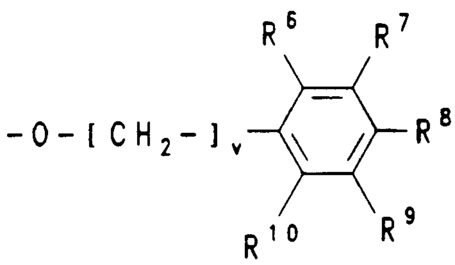

(V)

zu verstehen,

worin $R^6$, $R^7$, $R^8$ und $R^{10}$ gleich oder verschieden sind und Wasserstoff, Halogen, Cyano, Nitro, Trifluormethyl, ($C_1$-$C_6$)-Alkyl, ($C_1$-$C_6$)-Alkoxy, -O-$[CH_2-]_xC_fH_{(2f+1-g)}F_g$,-$OCF_2Cl$, -O-$CF_2$-CHFCl, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_4$)-Alkylcarbamoyl, N,N-Di-($C_1$-$C_4$)-alkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'R'', wie Amino, Anilino, N-Methylanilino, Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl bedeuten, oder zwei benachbarte Substituenten gemeinsam eine Kette -$[CH_2-]_n$ oder -CH=CH-CH=CH- bedeuten, wobei eine $CH_2$-Gruppe der Kette gegebenenfalls durch O, S, SO, $SO_2$ oder NR' ersetzt ist, sowie v = 1, 2, 3 oder 4, vorzugsweise 0 und 1 bedeutet.

Bevorzugt sind Verbindungen der allgemeinen Formel I in denen

$R^1$, $R^4$ und einer der beiden Reste $R^2$ oder $R^3$ gleich oder verschieden sind und Wasserstoff, Halogen, insbesondere Fluor, Chlor oder Brom, ($C_1$-$C_5$)-Alkyl, Hydroxy, ($C_1$-$C_5$)-Alkoxy, ($C_1$-$C_5$)-Alkoxycarbonyl, ($C_1$-$C_8$)-Alkylcarbonyloxy, -NR'-R'', ($C_1$-$C_4$)-Alkoxy-($C_1$-$C_6$)-alkyl, ($C_1$-$C_6$)-Alkoxy-($C_1$-$C_6$)-alkoxy oder einen substituierten ($C_6$-$C_{12}$)-Aryloxy- oder ($C_7$-$C_{11}$)-Aralkyloxyrest bedeuten, der 1, 2 oder 3 gleiche oder verschiedene Substituenten aus der Reihe, Halogen, insbesondere, Fluor oder Chlor, Cyano, Trifluormethyl, ($C_1$-$C_5$)-Alkyl, ($C_1$-$C_6$)-Alkoxy, ($C_1$-$C_5$)-Alkylcarbonyl, ($C_1$-$C_5$)-Alkoxycarbonyl, trägt, wobei
R' und R'' gleich oder verschieden sind und Wasserstoff, ($C_6$-$C_{12}$)-Aryl, ($C_1$-$C_8$)-Alkyl oder ($C_1$-$C_6$)-Alkylcarbonyl bedeutet,

n 0 oder 1 ist und

der andere Rest $R^2$ oder $R^3$ der allgemeinen Formel II entspricht, worin

X O,

Y $CH_2$-$OR^5$,

$R^6$ einen verzweigten oder unverzweigten ($C_1$-$C_8$)-Alkylrest, der einfach oder zweifach, insbesondere einfach substituiert ist mit Hydroxy, ($C_1$-$C_{10}$)-Alkoxy, ($C_1$-$C_8$)-Alkoxycarbonyl,($C_3$-$C_8$)-Cycloalkoxycarbonyl, ($C_7$-$C_{12}$)-Aralkoxycarbonyl, ($C_6$-$C_{10}$)-Aryloxycarbonyl, Carbamoyl, N-(($C_1$-$C_8$)-Alkoxy-($C_1$-$C_2$)alkyl)carbamoyl, N-(($C_1$-$C_4$)-Hydroxyalkyl)carbamoyl oder ein O-acyliertes Derivat hiervon, ($C_1$-$C_{12}$)-Alkylcarbonyloxy, Carboxyl, ($C_1$-$C_8$)-Alkoxycarbonyloxy, ($C_6$-$C_{12}$)-Aryloxycarbonyloxy, ($C_7$-$C_{11}$)-Aralkyloxycarbonyloxy, ($C_7$-$C_{11}$)-Aralkylcarbonyloxy, ($C_6$-$C_{12}$)-Arylcarbonyloxy, ($C_3$-$C_8$)-Cycloalkylcarbonyloxy, Carbamoyloxy, N-($C_1$-$C_6$)-Alkylcarbamoyloxy, N,N-Di-($C_1$-$C_8$)-alkylcarbamoyloxy, N-($C_3$-$C_8$)-Cycloalkylcarbamoyloxy, ($C_1$-$C_8$)-Alkylcarbonyl, ($C_3$-$C_8$)-Cycloalkylcarbonyl, NR'R'' oder dem Acylrest einer N-derivatisierten Aminosäure, oder mit
einem substituierten ($C_6$-$C_{12}$)Arylrest, der ein oder zwei Substituenten aus der Reihe Halogen, insbesondere Fluor, Chlor, Trifluormethyl, Hydroxy, ($C_1$-$C_4$)-Alkoxy, ($C_1$-$C_5$)-Alkyl, ($C_1$-$C_6$)-Alkylcarbonyloxy, ($C_1$-$C_6$)-Alkoxycarbonyl, ($C_1$-$C_4$)-Hydroxyalkyl aufweist, oder mit
einem substituierten ($C_6$-$C_{12}$)-Aryloxyrest, ($C_7$-$C_{11}$)-Aralkyloxyrest oder einem Heteroaryloxyrest, insbesondere einem Phenoxyrest oder einem Benzyloxyrest, der ein oder zwei Substituenten aus der Reihe Hydroxy, ($C_1$-$C_4$)-Alkoxy, ($C_1$-$C_5$)-Alkyl, ($C_1$-$C_6$)-Alkylcarbonyloxy, ($C_1$-$C_6$)-Alkoxycarbonyl, ($C_1$-$C_4$)-Hydroxyalkyl aufweist,
einen ($C_6$-$C_{12}$)-Arylrest, der 1, 2 oder 3-fach substituiert ist mit Hydroxy, Halogen, insbesondere Chlor oder Brom, Cyano, Nitro, ($C_1$-$C_6$)-Alkyl, ($C_1$-$C_6$)-Alkoxy, Trifluormethyl, ($C_1$-$C_6$)-Hydroxyalkyl, -O-$[CH_2]_xC_fH_{(2f+1g)}F_g$, -$OCF_2Cl$, $OCF_2$-CHFCl, ($C_1$-$C_6$)-Alkylcarbonyl, ($C_1$-$C_6$)-Alkoxycarbonyl, Carbamoyl, N-($C_1$-$C_8$)-Alkylcarbamoyl, N,N-Di-($C_1$-$C_8$)-alkylcarbamoyl, ($C_1$-$C_6$)-Alkylcarbonyloxy, Phenyl, Benzyl, Phenoxy, Benzyloxy und/oder NR'R'',

$R^7$ unabhängig von $R^6$ Wasserstoff oder ($C_1$-$C_5$)Alkyl sein kann,

$R^5$ H oder eine Alkohol-Schutzgruppe sein kann, wobei $OR^5$ insbesondere einen substituierten oder unsubstituierten Methylether, einen substituierten oder unsubstituierten Ethylether, einen substituierten oder unsubstituierten Benzylether, einen Silylether, einen Ester, ein Carbonat, ein Carbamat oder ein Sulfonat bedeutet.

Folgende Alkoholschutzgruppen können verwendet werden:

Als substituierte Methylether:

Methoxymethyl, Methylthiomethyl, t-Butylthiomethyl, (Phenyldimethylsilyl)methoxymethyl, Benzyloxymethyl, p-Methoxybenzyloxymethyl, (4-Methoxyphenoxy)-methyl, Guajacolmethyl, t-Butoxymethyl, 4-Pentenyloxymethyl, Siloxymethyl, 2-Methoxyethoxymethyl, 2,2,2-Trichlorethoxymethyl, Bis(2-chlorethoxy)methyl, 2-(Trimethylsilyl)ethoxymethyl, Tetrahydropyranyl, 3-Bromotetrahydropyranyl, Tetrahydrothiopyranyl, 1-Methoxycyclohexyl, 4-Methoxytetrahydropyranyl, 4-Methoxytetrahydrothiopyranyl, 4-Methoxytetrahydrothiopyranyl-S,S-Dioxo, 1-[2-Chlor-4-methyl)-phenyl]-4-methoxypiperidin-4-yl, 1,4-Dioxan-2yl, Tetrahydrofuranyl, Tetrahydrothiofuranyl.

Als substituierte Ethylether:

1-Ethoxyethyl, 1-(2-Chlorethoxy)ethyl, 1-Methyl-1-methoxyethyl, 1-Methyl-1-benzyloxyethyl, 1-Methyl-1-benzyloxy-2-fluoroethyl, 2,2,2-Trichlorethyl, 2-Trimethylsilylethyl, 2-(Phenylselenyl)ethyl, t-Butyl, Allyl, p-Chlorphenyl, p-Methoxyphenyl, 2,4-Dinitrophenyl, Benzyl.

Als substituierte Benzylether:

p-Methoxybenzyl, 3,4-Dimethoxybenzyl, o-Nitrobenzyl, p-Nitrobenzyl, p-Halogenbenzyl, 2,6-Dichlobenzyl, p-Cyanobenzyl, p-Phenylbenzyl, 2- und 4-Picolyl, 3-Methyl-2-picolyl-N-oxido, Diphenylmethyl, p,p'-Dinitrobenzhydryl, Triphenylmethyl, $\alpha$-Naphthyldiphenylmethyl, p-Methoxyphenyldiphenylmethyl, Di(p-methoxyphenyl)phenylmethyl, Tri(p-methoxyphenyl)methyl, 4-(4'-Bromphenacyloxy)phenyldiphenylmethyl, 4,4',4''-Tris(4,5-dichlorphthalimidophenyl)methyl, 4,4',4''-Tris(levulinooxyphenyl)methyl, 4,4',4''-Tris(benzoyloxyphenyl)methyl, 3-(Imidazol-1,4'-methyl)bis(4',4''-dimethoxyphenyl)methyl, 1,1-Bis(4-methoxyphenyl)-1'-pyrenylmethyl, 9-Anthryl, 9-(9-Phenyl)xanthenyl, 9-(9-Phenyl-10-oxo)anthryl.

Als Silylether:

Trimethylsilyl, Triethylsilyl, Triisopropylsilyl, Dimethylisopropylsilyl, Diethylisopropylsilyl, Dimethylthexylsilyl, t-Butyldimethylsilyl, t-Butyldiphenylsilyl, Tribenzylsilyl, Tri-p-xylylsilyl, Triphenylsilyl, Diphenylmethylsilyl, t-Butylmethoxyphenylsilyl.

Als Ester:

Formate, Benzoylformate, Aminosäureester, Acetate, Chloroacetat, Dichloroacetat, Trichloroacetat, Trifluoroacetat, Methoxyacetat, Triphenylmethoxyacetat, Phenoxyacetat, p-Chlorphenoxyacetat, p-P-Phenylacetat, 3-Phenylpropionat, 4-Oxopentanoat (Levulinat), 4,4-(Ethylendithio)pentanoat, Pivaloat, Adamantoat, Crotonat, 4-Methoxycrotonat, Benzoat, p-Phenylbenzoat, 2,4,6-Trimethylbenzoat (Mesitoat).

Als Carbonate:

Methyl, 9-Fluorenylmethyl, Ethyl, 2,2,2,-Trichlorethyl, 2-(Trimethylsilyl)ethyl, 2-(Phenylsulfonyl)ethyl, 2-(Triphenylphosphonio)ethyl, Isobutyl, Vinyl, Allyl, p-Nitrophenyl, Benzyl, p-Methoxybenzyl, 3,4-Dimethoxybenzyl, o-Nitrobenzyl, p-Nitrobenzyl, S-Benzyl Thioxarbonate, 4-Ethoxy-1-naphthyl, Methyl Dithiocarbonate.

Weitere Ester:

2,6-Dichlor-4-methylphenoxyacetat, 2,6-Dichlor-4-(1,1,3,3-tetramethylbutyl)phenoxyacetat, 2,4-Bis(1,1-dimethylpropyl)phenoxyacetat, Chlordiphenylacetat, Isobutyrat, Monosuccinat, (E)-2-Methyl-2-butenoat (Tigloat), O-(Methoxycarbonyl)benzoat, p-P-Benzoat, $\alpha$-Naphthoat, Nitrat, Alkyl N,N,N',N'-Tetramethylphosphorodiamidat, N-Phenylcarbamat, Borate, Dimethylphosphinothioyl, 2,4-Dinitrophenylsulfenat.

Als Sulfonate:

Sulfate, Methanesulfonat (Mesylat), Benzylsulfonat, Tosylate.

Insbesondere bevorzugt sind folgende Schutzgruppen als $R^5$:

$(C_1\text{-}C_6)$-Alkanoyl, $(C_1\text{-}C_8)$-Alkylcarbamoyl, Di-$(C_1\text{-}C_8)$-alkylcarbamoyl, N-$(C_3\text{-}C_8)$-cycloalkylcarbamoyl, $(C_1\text{-}C_6)$-Alkoxycarbonyl, $(C_6\text{-}C_{12})$-Aryloxycarbonyl, $(C_7\text{-}C_{11})$Aralkyloxycarbonyl, insbesondere Benzyloxycarbonyl, $(C_6\text{-}C_{12})$-Arylcarbonyl, $(C_7\text{-}C_{11})$-Aralkylcarbonyl, $(C_1\text{-}C_6)$-Alkyl, $(C_1\text{-}C_6)$-Alkoxy-$(C_1\text{-}C_6)$-alkyl, Carbamoyl-$(C_1\text{-}C_6)$-alkylester, $(C_1\text{-}C_{10})$-Acyloxy-$(C_1\text{-}C_6)$-alkyl, vorzugsweise $(C_1\text{-}C_{10})$-Alkanoyloxy-$(C_1\text{-}C_6)$-alkyl Benzyloxy-$(C_1\text{-}C_6)$-alkyl, Benzyloxycarbonyloxy-$(C_1\text{-}C_6)$-alkyl, $(C_1\text{-}C_6)$-Alkoxycarbonyloxy-$(C_1\text{-}C_6)$-alkyl, Phenyl, Benzyl, wobei der Phenyl gegebenenfalls substituiert sein kann, Aminosäureester oder Tetrahydropyranyl.

Aminoschutzgruppen sind in R. Geiger und W. König "The Peptides" Volume 3, "Protection of Functional Groups in Peptide Synthesis", E. G. Gross, J. Meienhöfer Edit, Academic Press, New York (1981), insbesondere Seiten 7-46, beschrieben.

Ebenso werden solche Gruppen in A. Hubbuch, Schutzgruppen in der Peptidsynthese, Kontakte 3/79. Seiten 19-23 beschrieben.

Insbesondere bevorzugt sind folgende Aminoschutzgruppen:

Acetamidomethyl

1-Adamantyloxycarbonyl
1-(1-Adamantyl)-1-methyl-ethoxycarbonyl
Allyloxycarbonyl
tert.-Butyloxycarbonyl
1-(4-Biphenylyl)-1-methyl-ethoxycarbonyl
Dicyclohexylcarbodiimid
$\alpha,\alpha$-Dimethyl-3,5-dimethoxybenzyloxycarbonyl
4-Dihydroxyborylbenzyloxycarbonyl
9-Fluorenylmethyloxycarbonyl
1-Hydroxybenzotriazol
3-Hydroxy-4-oxo-3,4-dihydro-1,2,3-benzotriazin
Isobornyloxycarbonyl
1-Methyl-cyclobutyloxycarbonyl
4-Methoxybenzyloxycarbonyl
Methylsulfonylethyloxycarbonyl
4-Pyridylmethyloxycarbonyl
2,2,2-Trichloro-tert.-butyloxycarbonyl
Benzyloxycarbonyl
halogensubstituiertes Benzyloxycarbonyl
4-Nitro-benzyloxycarbonyl
2-Phosphonmethyloxycarbonyl
Phenylsulfonylethoxycarbonyl
Toluolsulfonylethoxycarbonyl
2,3,5-Trimethyl-4-methoxy-phenylsulfonyl
Benzotriazol-1 yl-oxy-tris(dimethylamino)phosphonium-parafluorophosphat.

Ganz besonders bevorzugt ist $R^5$ Wasserstoff, ($C_1$-$C_8$)-Alkyl, Benzyl, ($C_1$-$C_6$)-Alkylcarbonyl, Phenylcarbonyl und die Carbamoylderivate, die oben genannt sind.

Besonders bevorzugt sind Verbindungen der Formel I, in denen Y = $CH_2OR^5$, n = 0 ist und

| | |
|---|---|
| $R^1$ und $R^4$ | sowie einer der Reste $R^2$ oder $R^3$ Wasserstoff, ($C_1$-$C_5$)-Alkyl, Fluor, Chlor, ($C_1$-$C_5$)-Alkoxy, und der andere Rest, $R^2$ oder $R^3$ der allgemeinen Formel II entspricht, worin |
| X | O, |
| $R^6$ | verzweigtes oder unverzweigtes ($C_1$-$C_3$)-Alkyl, welches einfach substituiert ist mit Hydroxy, ($C_1$-$C_4$)-Alkoxy, ($C_1$-$C_9$)-Alkoxycarbonyl, ($C_3$-$C_6$)-Cycloalkoxycarbonyl, Benzyloxycarbonyl, ($C_1$-$C_4$)-Alkylcarbonyloxy und/oder (((($C_1$-$C_9$)-Alkoxycarbonyl)methyl)-carbamoyl oder |
| $R^6$ | ($C_6$-$C_{12}$)Aryl, welches einfach oder zweifach substituiert ist mit Hydroxy, Halogen,($C_1$-$C_6$)-Alkoxy, ($C_1$-$C_6$)-Alkyl, Trifluormethyl, ($C_1$-$C_6$)-Hydroxyalkyl, $-O-[CH_2]_xC_fH_{(2f+1-g)}F_g$, und/oder NR'R'', bedeutet und |
| $R^7$ | Wasserstoff ist. |

Bevorzugt sind solche Verbindungen, in denen $R^6$ jeweils endständig substituiert ist.

Ganz besonders bevorzugt sind Verbindungen der allgemeinen Formel I, in denen $R^1$, $R^4$ sowie einer der beiden Reste $R^2$ oder $R^3$ Wasserstoff bedeuten. Folgende Verbindungen sind besonders anzuwenden.

6-Hydroxymethylpyridin-4-carbonsäure-(3-methoxypropyl)amid
6-Hydroxymethylpyridin-4-carbonsäure-(3-hydroxypropyl)amid
6-Methoxymethylpyridin-4-carbonsäure-(3-hydroxypropyl)amid
6-Hydroxymethylpyridin-4-carbonsäure-(2-hydroxyethyl)amid
6-Hydroxymethylpyridin-3-carbonsäure-(2-hydroxyethyl)amid

Weiterhin betrifft die Erfindung die Verwendung von Verbindungen der allgemeinen Formel I sowie die physiologisch verträglichen Salze zur Herstellung eines antifibrotisch wirksamen Arzneimittels.

Schließlich betrifft die Erfindung die Verbindungen der allgemeinen Formel I zur Verwendung als Arzneimittel.

Insbesondere betrifft die Erfindung die Verbindungen der Formel I zur Anwendung als Fibrosuppressiva und zur Beeinflussung des Stoffwechsels von Kollagen und kollagenähnlichen Stoffen.

Weiterhin betrifft die Erfindung Verfahren zur Herstellung von Verbindungen der allgemeinen Formel I.

Verbindungen der Formel I werden entweder

a) aus entsprechend substituierten Pyridin-4- (oder -5-)carbonsäuren oder ihren Estern der Formel VI (hier als 5-Carbonsäuren gezeigt) nach den bekannten Methoden der Amidsynthese (z.B. nach den Methoden der Peptidsynthese oder der Aminolyse von Estern), ggf. mit anschließender Oxidation zum Pyridin-N-oxid,

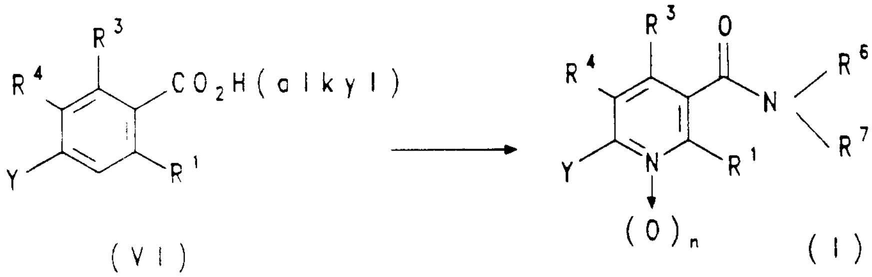

(VI) (I)

oder

b) aus Pyridin-4-(oder -5-)carbonsäureamidderivaten der Formel VII erhalten, in denen Y' ein Substituent bedeutet, der sich in einen Rest $-CH_2-OR^5$ umwandeln läßt, ggf. mit anschließender Oxidation zum Pyridin-N-oxid

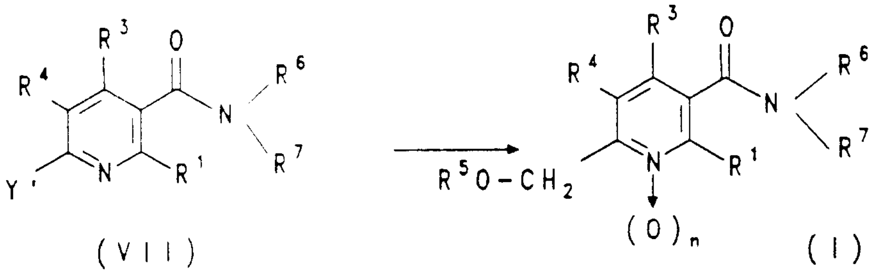

(VII) (I)

wobei

$b_1$) Verbindungen der Formel VII, in denen Y' = $CH_3$ und n = O bedeuten, zu Verbindungen der Formel VII halogeniert werden, in denen Y' = $-CH_2W$ (w = Halogen; Chlor, Brom) bedeutet und diese anschließend mit Reagenzien der Formel $MOR^5$ umgesetzt werden, worin M Wasserstoff, ein Alkali- oder ein Erdalkaliatom bedeutet, oder

$b_2$) Verbindungen der Formel VII, in denen Y' = $CH_3$ und n = 1 bedeuten, gemäß Schema 1 mit $Ac_2O/AcOH$ zu Verbindungen der Formel I umgesetzt werden, in denen Y = $-CH_2-OAc$ bedeutet, oder

$b_3$) Verbindungen der Formel VII, in denen Y' = $-CO_2H$, $-CO_2$-Alkyl oder - CHO und n = O bedeuten, durch den Fachmann bekannte Methoden reduziert werden zu Verbindungen der Formel I, in denen Y = $-CH_2OH$ bedeutet (vgl. Schema 2).

Das folgende Formelschema 1 gibt einen Überblick spezieller Verbindungen gemäß Formel I

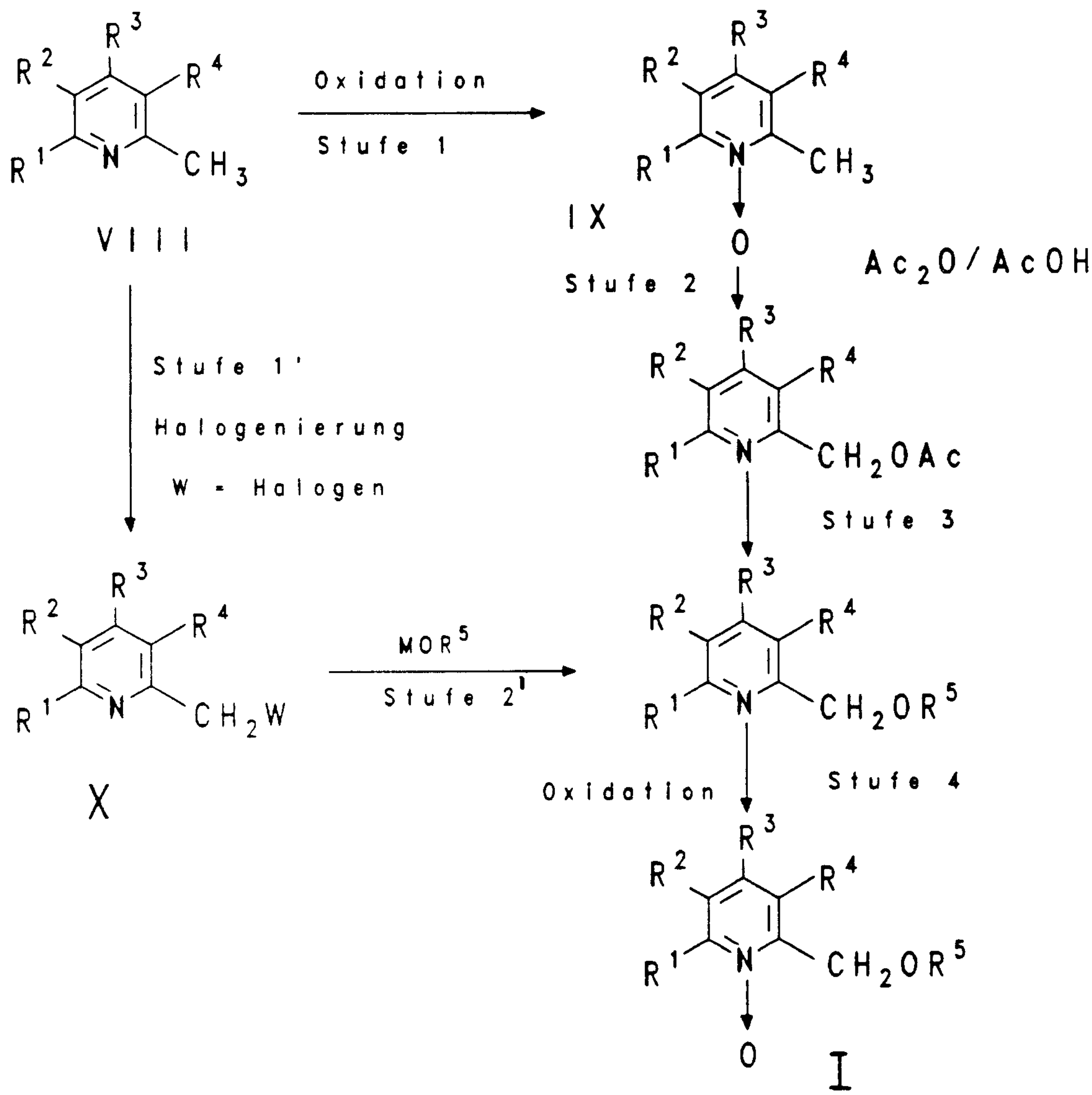


VIII IX X I

Gemäß Stufen 1 und 4 werden die Pyridinderivate VIII bzw. I in die entsprechenden Pyridin-N-oxide IX bzw. I überführt.

Diese Oxidation gelingt am einfachsten, in dem man Oxidationsmittel wie z.B. Wasserstoffperoxid oder Persäuren wie, Peressigsäure, Perfluoressigsaure, Perbenzoesäure oder Metachlorperbenzoesäure in Lösungsmitteln, wie chlorierte Kohlenstoffe, z.B. Methylenchlorid, Chloroform, Tri- oder Tetrachlorethylen, Benzol oder Toluol zu den zu oxidierenden Pyridinverbindungen, die ebenfalls in den obengenannten Lösungsmitteln gelöst sein können, gibt und bei Temperaturen zwischen -30 und +40 °C, bevorzugt 0 und +25 °C, zwischen 30 Minuten und 3 Tagen rührt. Das Ende der Reaktion läßt sich beispielsweise mittels Dünnschichtchromatographie bestimmen. Vorzugsweise lassen sich die erfindungsgemäßen Verbindungen I herstellen, in dem man das Pyridinderivat und das Oxidationsmittel in äquimolaren Mengen oder bis zu einem etwa 5-fachen Überschuß an Oxidationsmittel einsetzt.

Gegebenenfalls kann auch der Überschuß an Persäure beseitigt werden, in dem man beispielsweise gasförmig Ammoniak in die Reaktionslösung einleitet und den entstehenden Niederschlag durch Filtration von der Reaktionslösung abtrennt.

Gegebenenfalls kann die Aufarbeitung der Produkte beispielsweise durch Extraktion oder durch Chromatographie über Kieselgel erfolgen. Das isolierte Produkt kann umkristallisiert werden.

Eine allgemeine Vorschrift dieser Oxidationsmethode ist auch beispielsweise in "E. Klingsberg, Pyridine and its Derivatives, Interscience Publishers, New York, 1961, Part 2, 93" beschrieben.

Die Oxidation mit Wasserstoffperoxid ist beispielsweise in "E. Ochiai, J. Org. Chem. 18, 534 (1953)" beschrieben.

Ebenso können die erfindungsgemäßen Verbindungen mittels Oxidation gemäß den gleichzeitig eingereichten Deutschen Patentanmeldungen P 41 31 217.1 und P 41 31 220.1 hergestellt werden.

Die Darstellung der für die beschriebene Oxidation notwendigen unterschiedlichen Pyridinderivate wird in den schon als Stand der Technik zitierten Patentanmeldungen ausgeführt. Zu nennen sind die DE-A-38 26 471, 38 28 140, 39 24 093 und P 40 01 002.3 sowie die DE-A-37 03 959, 37 03 962 und 37 03 963.

Stufe 2 beschreibt die Umwandlung des Pyridin-N-oxides IX in die Pyridinverbindung I. Diese Umwandlung wird mitTrifluoracetanhydrid bei 0 °C oder mit Acetanhydrid, evtl. in Gegenwart einer Säure, wie z.B. Eisessig, bei 80-120 °C durchgeführt und man erhält die Acetate der Formel I. Die Umwandlung wird auch von Katritzky, A.R., Lagowski, J.M. in: "Chemistry of the Heterocyclic N-oxides", Academic Press: New York, 1972, IV. 2, Seiten 352-365, beschrieben.

Die Verseifung gemäß Stufe 3 wird nach bekannten Verfahren durchgeführt. Insbesondere werden hierzu Alkalihydroxide oder -carbonate in Methanol, Ethanol, Wasser oder ähnliches verwendet.

Stufe 1'

Verbindungen der Formel I, in denen $R^5$ Wasserstoff, n = 0 bedeutet und $R^1$ bis $R^4$ in der angegebenen Bedeutung stehen, können durch Halogenierung der entsprechenden 2-Picoline der Formel VIII mit N-Halogenamiden wie N-Bromsuccinimid oder N-Chlorphthalimid oder mit Trichlorisocyanursäure (vgl. Chem. Ber. 120, 649-651 (1987)) hergestellt werden.

Stufe 2'

Die Umsetzung der so erhaltenen 2-Picolylhalogenide der Formel X mit hydroxylhaltigen Salzen in der dem Fachmann geläufigen Art und Weise führt zu den erfindungsgemäßen Verbindungen der Formel I.

Auch durch Reaktion von Verbindungen der Formel I, in denen $OR^5$ eine sauerstoffhaltige Abgangsgruppe wie Tosylat, Brosylat, Mesylat oder Triflat und n = 0 bedeutet, mit hydroxyhaltigen Basen können die Titelverbindungen der Formel I mit $R^5$ = H hergestellt werden.

Die Herstellung von Derivaten, die auch am Pyridinring substituiert sein können, ist nachstehend in Formelschema 2 erläutert.

Pyridin-2,4-(und -2,5-)dicarbonsäurediester mit verschiedenen Substituenten $R^2$ ($R^3$) wie z.B. Halogen, Hydroxy, Alkoxy, Amino, 4-Butinyl-1-ol, Alkoxycarbonylalkyl, Hydroxyalkyl, 3-Propinyl-1-ol, sind aus der Anmeldung DE-A-37 07 429 bekannt.

Die selektive Verseifung der Estergruppe in 2-Position, beispielsweise die Cu-Salz vermittelte Esterspaltung von J. Delarge, Pharm. Acta Helv. 44, S. 637-643, 1969, erlaubt die Differenzierung der beiden Estergruppen und führt zu den Verbindungen der Formel XII. Die Aminolyse dieser Verbindungen führt zu den 4-Carbonsäureamiden der Formel XIII, deren Reduktion führt nach dem Fachmann geläufigen Reduktionsmethoden wie z.B. mit $BH_3 \cdot THF$, Boran-Tetrahydrofuran oder Boran-Dimethylsulfid-Komplex zu den Verbindungen der Formel I.

Folgendes Formelschema 2 dient zur Verdeutlichung für die Pyridin-4-carbonsäuren

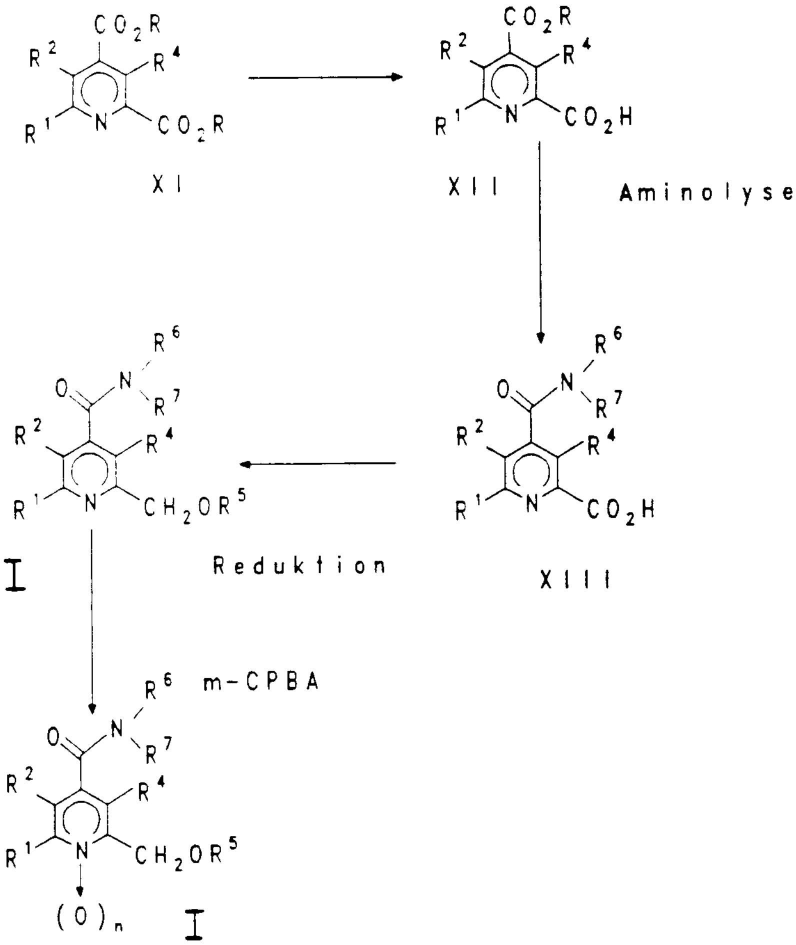


Die Verbindungen der Formel I, in denen $R^5$ = H und n = 0 bedeutet, können weiterhin durch Reduktion der entsprechenden Pyridin-2-aldehyde oder der Pyridin-2-carbonsäureester nach dem Fachmann geläufigen Methoden erhalten werden.

Geeignete Reduktionsmittel sind beispielsweise Lithiumaluminiumhydrid, Lithiumborhydrid, Natriumborhydrid und Diboran-Derivate.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum, Immunsuppressivum und Antiatherosklerotikum.

Die antifibrotische Wirkung kann im Modell der Tetrachlorkohlenstoff-induzierten Leberfibrose bestimmt werden. Dazu werden Ratten mit $CCl_4$ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen-$NC_1$) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von

a) unbehandelten Ratten (Kontrolle)

b) Ratten, denen Tetrachlorkohlenstoff verabreicht wurde ($CCl_4$-Kontrolle)

c) Ratten, denen zunächst $CCl_4$ und anschließend eine erfindungsgemäße Verbindung verabreicht wurde gemessen (diese Testmethode wird beschrieben von Rouiller, C., experimental toxic injury of the liver; in The Liver, C. Rouiller, Vol. 2, 5. 335-476, New York, Academic Press, 1964).

Ein anderes Modell zur Evaluierung der antibiotischen Wirkung ist das der Bleomycin-induzierten Lungenfibrose wie bei Kelley et al. (J. Lab. Clin. Med. 96, 954, (1980)) beschrieben. Für die Evaluierung der Wirkung der erfindungsgemäßen Verbindungen der Granulationsgewebe kann das Modell des Wattebauschgranuloms, wie bei Meier et al., Experimentia 6, 469 (1950) beschrieben, herangezogen werden.

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag, inbesondere 0,5 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die folgenden Beispiele sollen die Erfindung erläutern.

Unter anderen findet Methyl-2-hydroxymethyl-pyridin-4-carboxylat als eine Ausgangssubstanz Verwendung. Ihre Herstellung erfolgt wie von T. Sugiyama et al., Chem. Let. 1982, 917-920 beschrieben durch photochemische Hydroxymethylierung oder aus Methyl-2-methylpyridin-4-carboxylat, wie von J.K. Seydel et al. J. Med. Chem. 1976, 19, 483-492 beschrieben.

Beispiel 1:

6-Hydroxymethylpyridin-4-carbonsäure-(2-methoxyethyl)amid

10,0 g (59,8 mM) Methyl-2-hydroxymethylpyridin-4-carboxylat werden in 150 ml 2-Methoxyethylamin 5 h zum Rückfluß erhitzt. Man läßt auf Raumtemperatur abkühlen und destilliert überschüssiges Amin im Vakuum ab. Der Rückstand wird mit Ethylacetat/Methanol (5:1) an Kieselgel chromatographiert.

Es resultiert ein farbloses Öl, $R_f \approx 0,27$

Summenformel: $C_{10}H_{14}N_2O_3$

MS: m/e = 211,2 (M+H)

Beispiel 2:

6-Hydroxymethylpyridin-4-carbonsäure-(3-methoxypropyl)amid

Die Titelverbindung wird analog Beispiel 1 aus 1,67 g (10 mMol) Methyl-2-hydroxymethylpyridin-4-carboxylat und 20 ml 3-Methoxypropylamin hergestellt.

Es resultieren 1,7 g farbloses Öl, $R_f \approx 0,36$

Summenformel: $C_{11}H_{16}N_2O_3$

MS: m/e = 225,3 (M+H)

Beispiel 3:

6-Hydroxymethylpyridin-4-carbonsäure-(2-hydroxyethyl)amid

1,67 g (10 mM) Methyl-6-hydroxymethylpyridin-4-carboxylat werden in 15 ml 2-Aminoethanol 3 h bei 105-110 °C gerührt. Das überschüssige Amin wird i. Vak. abdestilliert und das Rohprodukt mit Ethylacetat/Methanol (5:1) an Kieselgel chromatographiert, ($R_f \approx 0{,}16$).

Es resultieren 2 g eines öligen Rohproduktes, das mit Ethylacetat zur Kristallisation gebracht wird, Fp. 81-82 °C.

Beispiel 4:

6-Hydroxymethylpyridin-4-carbonsäure-(3-hydroxypropyl)amid

Die Titelverbindung wird analog Beispiel 3 aus 10 g (59,8 mM) Methyl-6-hydroxymethylpyridin-4-carboxylat und 30 ml 1-Amino-3-propanol (4 h, 120-125 °C) hergestellt.

Nach Chromatrographie mit Ethylacetat/Methanol (5:1) resultieren 10,4 g eines farblosen öligen Produktes, $R_f \approx 0{,}16$.

Summenformel: $C_{10}H_{14}N_2O_3$

MS: m/e = 211 ($M^+$ + H)

Beispiel 5:

6-Hydroxymethylpyridin-4-carbonsäure-(2-methoxyethyl)amid-N-oxid

0,7 g (3,3 mM) der Titelverbindung aus Beispiel 1 werden in 50 ml Dichlormethan unter Rühren mit 1,2 g (4,95 mM) m-Chlorperbenzoesäure (70 %) versetzt. 3-Chlorbenzoesäure wird durch Einleiten von gasförmigem Ammoniak ausgefällt und abgesaugt. Das Filtrat wird eingeengt und mit Diisopropylether zur Kristallisation gebracht. Es konnten 0,66 g farblose Kristalle, Fp. 101-103 °C, gewonnen werden.

Summenformel: $C_{10}H_{14}N_2O_4$

MS: m/e = 227 ($M^+$ + H)

Beispiel 6:

6-Methoxymethylpyridin-4-carbonsäure-(2-methoxyethyl)amid

a) Unter Rühren werden zu einer Lösung von 1,5 g 6-Hydroxymethylpyridin-4-carbonsäuremethylester in 50 ml Dichlormethan unter Eiskühlung 3,0 ml Thionylchlorid $SOCl_2$, gelöst in 5 ml Dichlormethan, zugetropft. Nach Erwärmen auf Raumtemperatur wird noch 1 h gerührt, im Vakuum eingeengt, der Rückstand mit Diethylether behandelt und abgesaugt. Es konnten 1,8 g 6-Chlormethylpyridin-4-carbonsäuremethylester-hydrochlorid als farblose Kristalle gewonnen werden.

Fp. 116-118 °C

b) 2,2 g (10 mMol) der Verbindung von Beispiel 6a) werden in 50 ml wasserfreiem Methanol gelöst und bei 20 °C unter Rühren mit 3,6 ml (20 mMol) 5,5 molarer Natriummethylat-Lösung versetzt. Nachdem 6 h zum Rückfluß erhitzt wurde,läßt man auf 20 °C abkühlen und engt im Vakuum ein, versetzt mit Wasser und extrahiert dreimal mit Dichlormethan. Nach Abdestillieren des Lösungsmittels erhält man 1,0 g 2-Methoxymethylpyridin-4-carbonsäuremethylester als öliges Rohprodukt.

c) 0,9 g (50 mMol) der Verbindung von Beispiel 6b) werden in 20 ml 6-Methoxyethylamin 3 h zum Rückfluß erhitzt. Nach Abkühlen wird im Vakuum eingeengt und der ölige Rückstand mit Ethylacetat/Methanol (10:1) an Kieselgel chromatographiert. Es resultieren 0,85 g der Titelverbindung als farbloses Öl, $R_f$ = 0,40.

Summenformel: $C_{11}H_{16}N_2O_3$

MS: m/e = 225,3 ($M^+$ + H)

Beispiel 7:

6-Acetoxymethylpyridin-4-carbonsäure-(2-acetoxyethyl)amid

0,63 g (3,3 mMol) der Titelverbindung aus Beispiel 3 werden in 50 ml Dioxan-1,4 und 20 ml Dichiormethan suspendiert. Anschließend werden 0,1 g 4-N,N-Dimethylaminopyridin und 1,1 ml (8 mMol)

Triethylamin zugegeben. Dann tropft man 0,47 ml (6,6 mMol) Acetylchlorid zu und rührt 2 h bei 20 °C. Man engt ein, versetzt mit Wasser, extrahiert mit Dichlormethan, trocknet und befreit von Lösungsmittel. Es resultieren 0,21 g eines farblosen Öls.
$R_f$ = 0,40 (Ethylacetat/Methanol (5:1))
Summenformel: $C_{13}H_{16}N_2O_5$

MS: m/e = 281 ($M^+$ + H)

Beispiel 8:

6-Benzoyloxymethylpyridin-4-carbonsäure-(2-methoxyethyl)amid

Zu 0,7 g (3,3 mMol) der Titelverbindung aus Beispiel 1 in 50 ml Dichlormethan werden 100 mg 4-N,N-Dimethylaminopyridin und 0,54 ml (4 mMol) Triethylamin zugegeben. Nachdem 0,39 ml (3,3 mMol) Benzoylchlorid zugetropft wurden, rührt man 2 h bei 20 °C, engt im Vakuum ein, versetzt den Rückstand mit Wasser, extrahiert mit Dichlormethan, trocknet die organische Phase, dampft das Lösungsmittel im Vakuum ab und bringt den Rückstand mit Diethylether zur Kristallisation.
Es konnten 0,65 g farbloser Kristalle eines Schmelzpunkts Fp = 81-82 °C gewonnen werden.
Summenformel: $C_{17}H_{18}N_2O_4$

Beispiel 9:

6-Methoxymethyl-pyridin-4-carbonsäure-(3-methoxypropyl)amid

a) Zu 2,0 g (8,93 mM) der Titelverbindung von Beispiel 2 in 80 ml Dichlormethan werden unter Eiskühlung 6 ml Thionylchlorid in 20 ml Dichlormethan zugetropft. Nach 0,5 Stunden engt man im Vakuum ein, bringt den Rückstand mit Diethylether zur Kristallisation und erhält 2,3 g 6-Chlormethyl-pyridin-4-carbonsäure-(3-methoxypropyl)amid-hydrochlorid als farblose, kristalline Substanz, Fp. 128-130 °C.

b) 1,7 g (6,1 mM) der Verbindung aus 9a) werden in 50 ml Methanol und bei 20 °C mit 2,8 ml 5,5 molarer Natriummethylatlösung versetzt. Dann erhitzt man 5 Stunden zum Rückfluß, engt im Vakuum ein, versetzt mit 50 ml wäßriger Ammoniumchlorid-Lösung, extrahiert mit Dichlormethan, trocknet, engt ein und erhält 1,4 g der öligen Titelverbindung.

Summenformel: $C_{12}H_{18}N_2O_3$

MS: m/e = 239 ($M^+$ + H)

Beispiel 10:

6-Methoxymethylpyridin-4-carbonsäure-(3-hydroxypropyl)amid

2,7 g (14,9 mM) 6-Methoxymethylpyridin-4-carbonsäuremethylester (Verbindung aus Beispiel 6b) werden in 10 ml 3-Aminopropanol 1,5 Stunden auf 80 °C erwärmt. Nach Einengen im Vakuum und Chromatographieren mit Ethylacetat/Methanol (93:7) an Kieselgel erhält man die Titelverbindung als blaßgelbes Öl; $R_f$ ca. 0,17
Summenformel: $C_{11}H_{16}N_2O_3$

MS: m/e = 225 ($M^+$ + H)

Beispiel 11:

6-(N-Cyclohexylcarbamoyloxymethyl)-pyridin-4-carbonsäure-(3-methoxypropyl)amid

1,1 g (4,9 mM) 6-Hydroxymethylpyridin-4-carbonsäure (3-methoxypropyl)amid (Titelverbindung aus Beispiel 2) werden in 50 ml Dichlormethan bei 0 °C unter Rühren tropfenweise mit 3,5 ml (25 mM) Cyclohexylisocyanat versetzt und 2 Stunden zum Rückfluß erhitzt. Nach dem Abkühlen wird mit 20 ml Wasser versetzt und die organische Phase abgetrennt; nach Abdestillieren des Lösungsmittels erhält man ein öliges Rohprodukt, das mit Diethylether zur Kristallisation gebracht wird.

Es resultieren 1,3 g farblose Kristalle eines Schmelzpunkts von Fp = 117 - 119 °C.
Summenformel: $C_{18}H_{27}N_3O_4$

Beispiel 12:

6-Benzyloxymethylpyridin-4-carbonsäure-(3-methoxypropyl)amid

Die Titelverbindung kann analog Beispiel 6a) und b) aus 6-Chlormethylpyridin-4-carbonsäure-(3-methoxypropyl)amid-hydrochlorid, Benzylalkohol und Natrium hergestellt werden; farbloses Öl.
Summenformel: $C_{18}H_{22}N_2O_3$

MS: m/e = 315 ($M^+$ + H)

Beispiel 13:

6-Benzyloxymethylpyridin-4-carbonsäure-(2-methoxyethyl)amid

Die Herstellung erfolgt analog Beispiel 12.
Summenformel: $C_{17}$ $H_{20}$ $N_2$ $O_3$

MS: m/e = 301 ($M^+$ + H)

Beispiel 14

6-Benzyloxymethyl-pyridin-4-carbonsäure(2-hydroxyethyl)amid

a) 6-Chlormethylpyridin-4-carbonsäuremethylester-hydrochlorid

Unter Rühren werden zu einer Lösung von 16,7 g (0,01 mol 2-Hydroxymethylpyridin-4-carbonsäuremethylester in 500 ml Dichlormethan unter Eiskühlung 30 ml Thionylchlorid, gelöst in 70 ml Dichlormethan, zugetropft. Nach Erwärmen auf Raumtemperatur wird nach 1 h gerührt, im Vakuum eingeengt, der Rückstand mit Diethylether behandelt und 23 g des Produktes in Form farbloser Kristalle gewonnen, Fp. 116-118 °C.

b) 6-Benzyloxymethylpyridin-4-carbonsäure

60 ml (0,6 mol) Benzylalkohol werden in 500 ml N,N-Dimethylacetamid portionsweise mit 14,4 g (ca. 0,5 mol) 80 % Natriumhydrid versetzt. Man rührt 30 min bei 60 °C, kühlt auf 20 °C, gibt portionsweise 23 g (0,1 mol) der Verbindung aus Beispiel 2a zu, rührt 2 h bei 75 °C. Anschließend gibt man 300 ml Wasser zu, engt die dunke Lösung im Vakuum ein, gibt weitere 100 ml Wasser zu, versetzt mit 2N NaOH bis zur klaren Lösung, filtriert, und bringt das Fiftrat mit 2N HCl auf pH 4, saugt den Niederschlag ab, wäscht mit Wasser nach, trocknet und erhält 10,8 g Produkt, Fp. 173-175 °C.

c) 1,8 g (7,5 mmol) der vorstehenden Verbindung werden in 100 ml wasserfreiem Tetrahydrofuran gelöst und bei 0 °C unter Rühren mit 1,1 ml (8 mmol) Triethylamin und 0,8 ml (8 mmol) Chlorameisensäureethylester versetzt. Nach 30 min bei 0 °C tropft man 0,5 ml (8 mmol) Ethanolamin zu und rührt eine weitere Stunde bei 0 °C. Nach DC-Kontrolle wird eingeengt, 100 ml gesättigte wäßrige $NaHCO_3$-Lösung zugegeben, dreimal mit Dichlormethan ertrahiert, die organische Phase getrocknet und eingeengt. 2,5 g des öligen Rückstandes werden mit Ethylacetat an Kieselgel gereinigt, $R_f$ = 0,32.

Man erhält 1,8 g der Titelverbindung als farbloses Öl.
Summenformel: $C_{16}H_{18}N_2O_3$

MS: m/e = 287 ($M^+$ + H)

Beispiel 15

6-Methyloxymethyl-pyridin-3-carbonsäure-(2-hydroxyethyl)amid

a) 6-Methoxymethylpyridin-3-carbonsäuremethylester

52 g (0,28 mol) 6-Chlormethyl-pyridin-3-carbonsäuremethylester (hergestellt durch Chlorierung von 6-Methylpyridin-3-carbonsäuremethylester mit Trichlorisocyanursäure, vgl. Chem. Ber. 120, 649-651 (1987)) werden in 500 ml Methanol mit 54 ml (ca. 0,3 mol) 30 %iger Natriummethylat-Lösung und 4,5 g Natriumjodid versetzt und 3 h zum Rückfluß erhitzt. Das Reaktionsgemisch wird im Vakuum eingeengt, der Rückstand mit warmem Diisopropylether estrahiert und mit n-Heptan-Ethylacetat (2:1) an Kieselgel chromatographiert.

42,8 g Produkt, Fp. 34-36 °C (aus Petrolether)

b) 4,8 g (26,5 mmol) der vorstehenden Verbindung werden in 100 ml 2-Aminoethanol 5 h bei 50 °C gerührt. Man engt im Vakuum ein, chromatographiert den Rückstand mit Ethylacetat/Methanol (5:1) an Kieselgel und erhält 4,3 g der Titelverbindung als farblose Kristalle, Fp. 102-103 °C.

Summenformel: $C_{10}H_{14}N_2O_3$

MS: m/e = 211 ($M^+H$)

Beispiel 16

6-Hydroxymethyl-pyridin-3-carbonsäure(2-hydroxyethyl)amid

1 g (4,8 mmol) 6-Acetoxymethyl-pyridin-3-carbonsäure-methylester (hergestellt aus 6-Methyl-pyridin-3-carbonsäuremethylester-N-oxid und einer Mischung aus Eisessig/Acetanhydrid bei 110-115 ° C` Fp. 92-93 ° C (aus Diisopropylether)) werden in 5 ml 2-Aminoethanol 2 h bei 80 ° C gerührt. Überschüssiges Amin wird im Vakuum abdestilliert und der Rückstand mit Ethylacetat/Ethanol (10:1) zur Kristallisation gebracht. Man erhält 0,7 g der Titelverbindung Fp.: 131-133 ° C.
Summenformel: $C_9H_{12}N_2O_3$

MS: m/e = 197 ($M^+$ + H)

Beispiel 17

3-[((4-Ethyloxyphenyl)amino)carbonyl]-6-hydroxymethyl-pyridin

a) 6-Chlormethyl-pyridin-3-carbonsäure

2,8 g (15,0 mmol) 6-Chlormethyl-pyridin-3-carbonsäure-methylester (hergestellt durch Chlorierung von 6-Methyl-pyridin-3-carbonsäure-methylester mit Trichlorisocyanursäure) werden in 100 ml 1,4-Dioxan mit 0,6 g (15 mmol) NaOH, gelöst in 20 ml Wasser, versetzt und 2 h bei 20 ° C gerührt, DC-Kontrolle. Man gibt weitere 5 ml 1 N wäßrige NaOH zu, rührt 0,5 h bei 20 ° C, engt im Vakuum ein, saugt ab, wäscht den Niederschlag mit Wasser, trocknet und erhält 1,7 g Produkt, Fp. 159-161 ° C.

Das wäßrige Filtrat wird zweimal mit Ethylacetat extrahiert, die organische Phase getrocknet, eingeengt und der Rückstand mit Diisopropylether/Ethylacetat (4:1) zur Kristallisation gebracht. Man erhält weitere 0,8 g Produkt, Fp. 159-161 ° C.

b) 3-[(4-Ethyloxyphenylamino)carbonyl]-6-chlormethyl-pyridin

2,6 g (15 mmol) der vorstehenden Verbindung werden in 100 ml wasserfreiem Tetrahydrofuran gelöst und bei -5 ° C mit 2,4 ml (17 mmol) Triethylamin versetzt. Nach 0,5 h tropft man 2,1 ml (17 mmol) Pivaloylchlorid, nach einer weiteren Stunde bei dieser Temperatur 2,2 ml (17 mmol) p-Phenetidin zu, rührt 1 h bei 0 ° C und läßt auf 25 ° C erwärmen.

Man engt im Vakuum ein, versetzt mit Wasser und saugt die ausgefallene Substanz ab. Diese wird in Dichlormethan gelöst, mit 2N wäßriger HCl und dann mit gesättigter wäßriger $NaHCO_3$-Lösung geschüttelt. Anschließend wird getrocknet und Dichlormethan im Vakuum abdestilliert. Der Rückstand wird mit 100 ml Ethylacetat bei 20 ° C behandelt, das kristalline Produkt abgesaugt und mit Ethylacetat gewaschen und getrocknet, 1,4, Fp. 172-174 ° C.

c) Die Titelverbindung wird erhalten, indem 0,9 g (3 mmol) der vorstehenden Verbindung in 50 ml 1,4-Dioxan unter Rühren mit 20 ml 1n wäßriger NaOH-Lösung 3 h auf 60 ° C erwärmt werden. Nach dem Abkühlen engt man im Vakuum ein, versetzt den Rückstand mit wäßriger 2n Salzsäure, saugt den kristallinen Niederschlag ab, trocknet und erhält 0,6 g Rohprodukt, Fp. ca. 220 ° C, daß weiß mit Aceton/Tetrahydrofuran (1:1) behandelt wird. Man erhält 0,4 g Produkt, Fp. 234-235 ° C. Weiterhin kann aus Methanol oder N,N-Dimethylformamid umkristallisiert werden, Fp. 236-238 ° C.

Beispiel 18

3-[(4-Methoxyphenylamino)carbonyl]-6-hydroxymethyl-pyridin

Beispiel 19

3-[(4-n-Hexyloxyphenylamino)carbonyl]-6-hydroxymethyl-pyridin

Beispiel 20

3-[(4-(4-Fluorphenoxy)phenylamino)carbonyl]-6-hydroxymethyl-pyridin

Beispiel 21

3-[(2-Methoxyphenylamino)carbonyl]-6-hydroxymethyl-pyridin

Beispiel 22

3-[(4-n-Butylphenylamino)carbonyl]-6-hydroxymethyl-pyridin

Beispiel 23

3-[((3-Trifluormethyl)phenylamino)carbonyl]-6-hydroxymethyl-pyridin

Beispiel 24

3-[(4-Chlorphenylamino)carbonyl]-6-hydroxymethyl-pyridin

Beispiel 25

3-[(4-Fluorphenylamino)carbonyl]-6-hydroxymethyl-pyridin

Beispiel 26

3-[(2-Fluorphenylamino)carbonyl]-6-hydroxymethyl-pyridin

Beispiel 27

3-[(Phenylamino)carbonyl]-6-hydroxymethyl-pyridin

Beispiel 28

3-[(3-Chlorphenylamino)carbonyl]-6-hydroxymethyl-pyridin

Beispiel 29

3-[(4-(N,N-Dimethylamino)phenylamino)carbonyl]-6-hydroxymethyl-pyridin

Beispiel 30

3-[(4-(2,2,2-Trifluorethyloxy)phenylamino)carbonyl]-6-hydroxymethyl-pyridin

Beispiel 31

3-[(4-((2,2,3,3,4,4,4-Heptafluorbutyl)oxy)phenylamino)carbonyl]-6-hydroxymethyl-pyridin

Beispiel 32

3-[(4-Ethyloxyphenylamino)carbonyl]-6-methyloxy-methyl-pyridin

Beispiel 33

3-[(4-n-Hexyloxyphenylamino)carbonyl]-6-methyloxy-methyl-pyridin

Beispiel 34

3-[(4-(4-Fluorphenoxy)phenylamino)carbonyl]-6-methyloxy-methyl-pyridin

Beispiel 35

3-[(2-Methoxyphenylamino)carbonyl]-6-methyloxymethyl-pyridin

Beispiel 36

3-[(4-n-Butylphenylamino)carbonyl]-6-methyloxymethyl-pyridin

Beispiel 37

3-[((3-Trifluormethyl)phenylamino)carbonyl]-6-methyloxymethyl-pyridin

Beispiel 38

3-[(4-Chlorphenylamino)carbonyl]-6-methyloxymethyl-pyridin

Beispiel 39

3-[(4-Fluorphenylamino)carbonyl]-6-methyloxymethyl-pyridin

Beispiel 40

3-[(2-Fluorphenylamino)carbonyl]-6-methyloxymethyl-pyridin

Beispiel 41

3-[(Phenylamino)carbonyl]-6-methyloxymethyl-pyridin

Beispiel 42

3-[(3-Chlorphenylamino)carbonyl]-6-methyloxymethyl-pyridin

Beispiel 43

3-[(4-(N,N-Dimethylamino)phenylamino)carbonyl]-6-methyloxymethyl-pyridin

Beispiel 44

3-[(4-((2,2,2-Trifluorethyl)oxy)phenylamino)carbonyl]-6-methyloxymethyl-pyridin

Beisiel 45

3-[(4-((2,2,3,3,4,4,4-Heptafluorbutyl)oxy)phenylamino)carbonyl]-6-methyloxymethyl-pyridin

Beispiel 46

3-[(4-Ethyloxyphenylamino)carbonyl]-6-ethyloxy-methyl-pyridin

Beispiel 47

3-[(4-n-Hexyloxyphenylamino)carbonyl]-6-ethyloxymethyl-pyridin

Beispiel 48

3-[(4-(4-Fluorphenoxy)phenylamino)carbonyl]-6-ethyloxymethyl-pyridin

Beispiel 49

3-[(2-Methoxyphenylamino)carbonyl]-6-ethyloxymethyl-pyridin

Beispiel 50

3-[(4-n-Butylphenylamino)carbonyl]-6-ethyloxymethyl-pyridin

Beispiel 51

3-[((3-Trifluormethyl)phenylamino)carbonyl]-6-ethyloxymethyl-pyridin

Beispiel 52

3-[(4-Chlorphenylamino)carbonyl]-6-ethyloxymethyl-pyridin

Beispiel 53

3-[(4-Fluorphenylamino)carbonyl]-6-ethyloxymethyl-pyridin

Beispiel 54

3-[(2-Fluorphenylamino)carbonyl]-6-ethyloxymethyl-pyridin

Beispiel 55

3-[(Phenylamino)carbonyl]-6-ethyloxymethyl-pyridin

Beispiel 56

3-[(3-Chlorphenylamino)carbonyl]-6-ethyloxymethyl-pyridin

Beispiel 57

3-[(4-(N,N-Dimethylamino)phenylamino)carbonyl]-6-ethyloxymethyl-pyridin

Beispiel 58

3-[(4-(2,2,2-Trifluorethyloxy)phenylamino)carbonyl]-6-ethyloxymethyl-pyridin

Beispiel 59

3-[(4-((2,2,3,3,4,4,4-Heptafluorbutyl)oxy)phenylamino)carbonyl]-6-ethyloxymethyl-pyridin

Beispiel 60

3-[(4-Ethyloxyphenylamino)carbonyl]-6-benzyloxymethyl-pyridin

Beispiel 61

3-[(4-n-Hexyloxyphenylamino)carbonyl]-6-benzyloxymethyl-pyridin

Beispiel 62

3-[(4-(4-Fluorphenoxy)phenylamino)carbonyl]-6-benzyloxymethyl-pyridin

Beispiel 63

3-[(2-Methoxyphenylamino)carbonyl]-6-benzyloxymethyl-pyridin

Beispiel 64

3-[(4-n-Butylphenylamino)carbonyl]-6-benzyloxymethyl-pyridin

Beispiel 65

3-[((3-Trifluormethyl)phenylamino)carbonyl]-6-benzyloxymethyl-pyridin

Beispiel 66

3-[(4-Chlorphenylamino)carbonyl]-6-benzyloxymethyl-pyridin

Beispiel 67

3-[(4-Fluorphenylamino)carbonyl]-6-benzyloxymethyl-pyridin

Beispiel 68

3-[(2-Fluorphenylamino)carbonyl]-6-benzyloxymethyl-pyridin

Beispiel 69

3-[(Phenylamino)carbonyl]-6-benzyloxymethyl-pyridin

Beispiel 70

3-[(3-Chlorphenylamino)carbonyl]-6-benzyloxymethyl-pyridin

Beispiel 71

3-[(4-(N,N-Dimethylamino)phenylamino)carbonyl]-6-benzyloxymethyl-pyridin

Beispiel 72

3-[(4-(2,2,2-Trifluorethyloxy)phenylamino)carbonyl]-6-benzyloxymethyl-pyridin

Beispiel 73

3-[(4-((2,2,3,3,4,4,4-Heptafluorbutyl)oxy)phenylamino)carbonyl]-6-benzyloxymethyl-pyridin

Beispiel 74

3-[(4-Ethyloxyphenylamino)carbonyl]-6-acetoxymethyl-pyridin

Beispiel 75

3-[(4-n-Hexyloxyphenylamino)carbonyl]-6-acetoxymethyl-pyridin

Beispiel 76

3-[(4-(4-Fluorphenoxy)phenylamino)carbonyl]-6-acetoxymethyl-pyridin

Beispiel 77

3-[(2-Methoxyphenylamino)carbonyl]-6-acetoxymethyl-pyridin

Beispiel 78

3-[(4-n-Butylphenylamino)carbonyl]-6-acetoxymethyl-pyridin

Beispiel 79

3-[((3-Trifluormethyl)phenylamino)carbonyl]-6-acetoxymethyl-pyridin

Beispiel 80

3-[(4-Chlorphenylamino)carbonyl]-6-acetoxymethyl-pyridin

Beispiel 81

3-[(4-Fluorphenylamino)carbonyl]-6-acetoxymethyl-pyridin

Beispiel 82

3-[(2-Fluorphenylamino)carbonyl]-6-acetoxymethyl-pyridin

Beispiel 83

3-[(Phenylamino)carbonyl]-6-acetoxymethyl-pyridin

Beispiel 84

3-[(3-Chlorphenylamino)carbonyl]-6-acetoxymethyl-pyridin

Beispiel 85

3-[(4-(N,N-Dimethylamino)phenylamino)carbonyl]-6-acetoxymethyl-pyridin

Beispiel 86

3-[(4-(2,2,2-Trifluorethyloxy)phenylamino)carbonyl]-6-acetoxymethyl-pyridin

Beispiel 87

3-[(4-((2,2,3,3,4,4,4-Heptafluorbutyl)oxy)phenylamino)carbonyl]-6-acetoxymethyl-pyridin

Beispiel 88

3-[(4-Ethyloxyphenylamino)carbonyl]-6-benzoyloxymethyl-pyridin

Beispiel 89

3-[(4-n-Hexyloxyphenylamino)carbonyl]-6-benzoyloxymethyl-pyridin

Beispiel 90

3-[(4-(4-Fluorphenoxy)phenylamino)carbonyl]-6-benzoyloxymethyl-pyridin

Beispiel 91

3-[(2-Methoxyphenylamino)carbonyl]-6-benzoyloxymethyl-pyridin

Beispiel 92

3-[(4-n-Butylphenylamino)carbonyl]-6-benzoyloxymethyl-pyridin

Beispiel 93

3-[((3-Trifluormethyl)phenylamino)carbonyl]-6-benzoyloxymethyl-pyridin

Beispiel 94

3-[(4-Chlorphenylamino)carbonyl]-6-n-butyloxymethyl-pyridin

Beispiel 95

3-[(4-Fluorphenylamino)carbonyl]-6-n-butyloxymethyl-pyridin

Beispiel 96

3-[(2-Fluorphenylamino)carbonyl]-6-n-buryloxymethyl-pyridin

Beispiel 97

3-[(Phenylamino)carbonyl]-6-n-butyloxymethyl-pyridin

Beispiel 98

3-[(3-Chlorphenylamino)carbonyl]-6-n-butyloxymethyl-pyridin

Beispiel 99

3-[(4-(N,N-Dimethylamino)phenylamino)carbonyl]-6-n-butyloxymethyl-pyridin

Beispiel 100

3-[(4-(,2,2,2-Trifluorethyloxy)phenylamino)carbonyl]-6-(2-propyloxy)methyl-pyridin

Beispiel 101

3-[(4-((2,2,3,3,4,4,4-Heptafluorbutyl)oxy)phenylamino)carbonyl]-6-(2-propyloxy)-methyl-pyridin

Beispiel 102

3-[(4-Ethyloxyphenylamino)carbonyl]-6-cyclohexanoyloxymethyl-pyridin

Beispiel 103

3-[(4-n-Hexyloxyphenylamino)carbonyl]-6-(2-methylbenzoyl)methyl-pyridin

Beispiel 104

3-[(4-(4-Fluorphenoxy)phenylamino)carbonyl]-6-(2-methylbenzoyl)methyl-pyridin

Beispiel 105

3-[(2-Methoxyphenylamino)carbonyl]-6-(2-methylpropionyloxy)methyl-pyridin

Beispiel 106

3-[(4-n-Butylphenylamino)carbonyl]-6-propionyloxymethyl-pyridin

Beispiel 107

3-[((3-Trifluormethyl)phenylamino)carbonyl]-6-propionyloxymethyl-pyridin

Beispiel 108

3-[(4-Chlorphenylamino)carbonyl]-6-(2-methylpropionyl)oxymethyl-pyridin

Beispiel 109

3-[(4-Fluorphenylamino)carbonyl]-6-(2-methylpropionyl)oxymethyl-pyridin

Beispiel 110

3-[(2-Fluorphenylamino)carbonyl]-6-(2-methylpropionyl)oxy-pyridin

Beispiel 111

3-[(Phenylamino)carbonyl]-6-(2-methylbenzoyl)oxymethyl-pyridin

Beispiel 112

3-[(3-Chlorphenylamino)carbonyl]-6-(2-methylpropionyl)oxymethyl-pyridin

Beispiel 113

3-[(4-(N,N-Dimethylamino)phenylamino)carbonyl]-6-butanoyloxymethyl-pyridin

Beispiel 114

3-[(4-(2,2,2-Trifluorethyloxy)phenylamino)carbonyl]-6-butanoyloxymethyl-pyridin

Beispiel 115

3-[(4-((2,2,3,3,4,4,4-Heptafluorbutyl)oxy)phenylamino)carbonyl]-6-butanoyloxy-methyl-pyridin

Beispiel 116

6-Benzyloxymethyl-pyridin-4-carbonsäure-(glycylmethylester)amid

3,6 g (15 mmol) 6-Benzyloxymethyl-pyridin-4-carbonsäure werden in 80 ml Acetonitril suspendiert und mit 1,9 g (15 mmol) Glycinmethylester-Hydrochlorid, 3,8 ml (30 mmol) N-Ethylmorpholin, 2,2 g (16,5 mmol) 1-Hydroxy-1H-benzotriazol-Hydrat und 3,1 g (15 mmol) N,N'-Dicyclohexylcarbodiimid 20 h bei 20°C gerührt. Man saugt vom Ungelösten ab, wäscht mit Acetonitril, engt das Filtrat im Vakuum ein, nimmt den Rückstand in Dichlormethan auf, extrahiert die organische Phase mit gesättigter wäßriger $NaHCO_3$-Lösung, anschließend mit wäßriger $NH_4Cl$-Lösung, trocknet die organische Phase, engt ein und chromatographiert den Rückstand (4,5 g) mit Ethylacetat an Kieselgel. 3,3 g der Titelverbindung werden als farbloses Öl erhalten, $R_f$ = 0,5.
Summenformel: $C_{17}H_{18}N_2O_4$

MS: m/e = 315 ($M^+$ + H).

Beispiel 117

6-Methyloxymethyl-pyridin-3-carbonsäure-(glycyibenzylester)amid

a) 6-Methyloxymethyl-pyridin-3-carbonsäure

6,2 g (34,2 mmol) 6-Methyloxymethyl-pyridin-3-carbonsäure-methylester (vgl. Beispiel 15a) werden in 200 ml Methanol und 34,3 ml 1n wäßriger Natronlauge verseift. Nach Einengen, Ansäuern mit Salzsäure wird mit Ethylacetat ertrahiert, Ausbeute 4,3 g.

b) 4,3 g (25,7 mmol) der vorstehenden Verbindung werden in 100 ml wasserfreiem Tetrahydrofuran bei 5°C mit 7,9 ml (56,5 mmol) Triethylamin versetzt. Nach 20 min tropft man 3,47 ml (28,6 mmol) Pivaloylchlorid zu und rührt 2 h weiter bei 0-5°C. Dann gibt man 9,65 g (28,6 mmol) Glycinbenzylester-Tosylat zu, rührt 30 min bei 0°C, läßt auf 20°C erwärmen, rührt 16 h, gibt 400 ml Ethylacetat zu, wäscht mit Wasser, anschließend mit gesättigter $NaHCO_3$-Lösung, trocknet und chromatographiert den Rückstand mit Ethylacetat/n-Heptan (3:1) an Kieselgel. Man erhält 2 g der Titelverbindung als farblose Kristalle, Fp. 75-76°C.

Summenformel: $C_{17}H_{18}N_2O_4$

MS: m/e = 315 ($M^+$ + H).

Beispiel 118

6-Ethyloxymethyl-pyridin-3-carbonsäure-(glycylethylester) amid

Beispiel 119

6-Ethyloxymethyl-pyridin-3-carbonsäure-(glycyl-(2-propyl)ester)amid

Beispiel 120

6-Ethyloxymethyl-pyridin-3-carbonsäure-(glycyl-(3-pentyl)ester)amid

Beispiel 121

6-Ethyloxymethyl-pyridin-3-carbonsäure-(glycyl-(4-heptyl)ester)amid

Beispiel 122

6-Ethyloxymethyl-pyridin-3-carbonsäure-(glycyl-(5-nonyl)ester)amid

Beispiel 123

6-Ethyloxymethyl-pyridin-3-carbonsäure-(glycyl-(2,5-dimethyl-3-pentyl)ester)amid

Beispiel 124

6-Ethyloxymethyl-pyridin-3-carbonsäure-(glycyl-(3-methyl-3-pentyl)ester)amid

Beispiel 125

6-Ethyloxymethyl-pyridin-3-carbonsäure-(glycyl-((R)-2-butyl)ester)amid

Beispiel 126

6-Ethyloxymethyl-pyridin-3-carbonsäure-(glycyl-((S)-2-butyl)ester)amid

Beispeil 127

6-Ethyloxymethyl-pyridin-3-carbonsäure-(glycyl-cyclohexyl)ester)amid

Beispiel 128

6-Ethyloxymethyl-pyridin-3-carbonsäure-(glycyl-cyclopentyl)ester)amid

Beispiel 129

6-Ethyloxymethyl-pyridin-3-carbonsäure-(glycyl-glycylmethylester)amid

Beispiel 130

6-Ethyloxymethyl-pyridin-3-carbonsäure-(glycyl-glycyl-(2-propyl)ester)amid

Beispiel 131

6-Acetoxymethyl-pyridin-3-carbonsäure-(glycyl-ethylester) amid

Beispiel 132

6-Acetoxymethyl-pyridin-3-carbonsäure-(glycyl-(2-propyl)ester)amid

Beispiel 133

6-Acetoxymethyl-pyridin-3-carbonsäure-(glycyl-(3-pentyl)ester)amid

Beispiel 134

6-Acetoxymethyl-pyridin-3-carbonsäure-(glycyl-(4-heptyl)ester)amid

Beispiel 135

6-Acetoxymethyl-pyridin-3-carbonsäure-(glycyl-(5-nonyl)ester)amid

Beispiel 136

6-Acetoxymethyl-pyridin-3-carbonsäure-(glycyl-(2,5-dimethyl-3-pentyl)ester)amid

Beispiel 137

6-Acetoxymethyl-pyridin-3-carbonsäure-(glycyl-(3-methyl-3-pentyl)ester)amid

Beispiel 138

6-Acetoxymethyl-pyridin-3-carbonsäure-(glycyl-((R)-2-butyl)ester)amid

Beispiel 139

6-Acetoxymethyl-pyridin-3-carbonsäure-(glycyl-((S)-2-butyl)ester)amid

Beispeil 140

6-Acetoxymethyl-pyridin-3-carbonsäure-(glycyl-cyclohexyl)ester)amid

Beispiel 141

6-Acetoxymethyl-pyridin-3-carbonsäure-(glycyl-cyclopentyl)ester)amid

Beispiel 142

6-Acetoxymethyl-pyridin-3-carbonsäure-(glycyl-glycylmethylester)amid

Beispiel 143

6-Acetoxymethyl-pyridin-3-carbonsäure-(glycyl-glycyl-(2-propyl)ester)amid

Beispiel 144

6-Hydroxymethyl-pyridin-3-carbonsäure-(glycylethylester) amid

Beispiel 145

6-Hydroxymethyl-pyridin-3-carbonsäure-(glycyl-(2-propyl)ester)amid

Beispiel 146

6-Hydroxymethyl-pyridin-3-carbonsäure-(glycyl-(3-pentyl)ester)amid

Beispiel 147

6-Hydroxymethyl-pyridin-3-carbonsäure-(glycyl-(4-heptyl)ester)amid

Beispiel 148

6-Hydroxymethyl-pyridin-3-carbonsäure-(glycyl-(5-nonyl)ester)amid

Beispiel 149

6-Hydroxymethyl-pyridin-3-carbonsäure-(glycyl-(2,5-dimethyl-3-pentyl)ester)amid

Beispiel 150

6-Hydroxymethyl-pyridin-3-carbonsäure-(glycyl-(3-methyl-3-pentyl)ester)amid

Beispiel 151

6-Hydroxymethyl-pyridin-3-carbonsäure-(glycyl-((R)-2-butyl)ester)amid

Beispiel 152

6-Hydroxymethyl-pyridin-3-carbonsäure-(glycyl-((S)-2-butyl)ester)amid

Beispeil 153

6-Hydroxymethyl-pyridin-3-carbonsäure-(glycyl-cyclohexyl)ester)amid

Beispiel 154

6-Hydroxymethyl-pyridin-3-carbonsäure-(glycyl-cyclopentyl)ester)amid

Beispiel 155

6-Hydroxymethyl-pyridin-3-carbonsäure-(glycyl-glycylmethylester)amid

Beispiel 156

6-Hydroxymethyl-pyridin-3-carbonsäure-(glycyl-glycyl-(2-propyl)ester)amid

Beispiel 157

6-Acetoxymethyl-pyridin-4-carbonsäure-(glycyl-ethylester) amid

Beispiel 158

6-Acetoxymethyl-pyridin-4-carbonsäure-(glycyl-(2-propyl)ester)amid

Beispiel 159

6-Acetoxymethyl-pyridin-4-carbonsäure-(glycyl-(3-pentyl)ester)amid

Beispiel 160

6-Acetoxymethyl-pyridin-4-carbonsäure-(glycyl-(4-heptyl)ester)amid

Beispiel 161

6-Acetoxymethyl-pyridin-4-carbonsäure-(glycyl-(5-nonyl)ester)amid

Beispiel 162

6-Acetoxymethyl-pyridin-4-carbonsäure-(glycyl-(2,5-dimethyl-3-pentyl)ester)amid

Beispiel 163

6-Acetoxymethyl-pyridin-4-carbonsäure-(glycyl-(3-methyl-3-pentyl)ester)amid

Beispiel 164

6-Acetoxymethyl-pyridin-4-carbonsäure-(glycyl-((R)-2-butyl)ester)amid

Beispiel 165

6-Acetoxymethyl-pyridin-4-carbonsäure-(glycyl-((S)-2-butyl)ester)amid

Beispeil 166

6-Acetoxymethyl-pyridin-4-carbonsäure-(glycyl-cyclohexyl)ester)amid

Beispiel 167

6-Acetoxymethyl-pyridin-4-carbonsäure-(glycyl-cyclopentyl)ester)amid

Beispiel 168

6-Acetoxymethyl-pyridin-4-carbonsäure-(glycyl-glycylmethylester)amid

Beispiel 169

6-Acetoxymethyl-pyridin-4-carbonsäure-(glycyl-glycyl-(2-propyl)ester)amid

Beispiel 170

6-Hydroxymethyl-pyridin-4-carbonsäure-(glycylethylester) amid

Beispiel 171

6-Hydroxymethyl-pyridin-4-carbonsäure-(glycyl-(2-propyl)ester)amid

Beispiel 172

6-Hydroxymethyl-pyridin-4-carbonsäure-(glycyl-(3-pentyl)ester)amid

Beispiel 173

6-Hydroxymethyl-pyridin-4-carbonsäure-(glycyl-(4-heptyl)ester)amid

Beispiel 174

6-Hydroxymethyl-pyridin-4-carbonsäure-(glycyl-(5-nonyl)ester)amid

Beispiel 175

6-Hydroxymethyl-pyridin-4-carbonsäure-(glycyl-(2,5-dimethyl-3-pentyl)ester)amid

Beispiel 176

6-Hydroxymethyl-pyridin-4-carbonsäure-(glycyl-(3-methyl-3-pentyl)ester)amid

Beispiel 177

6-Hydroxymethyl-pyridin-4-carbonsäure-(glycyl-((R)-2-butyl)ester)amid

Beispiel 178

6-Hydroxymethyl-pyridin-4-carbonsäure-(glycyl-((S)-2-butyl)ester)amid

Beispeil 179

6-Hydroxymethyl-pyridin-4-carbonsäure-(glycyl-cyclohexyl)ester)amid

Beispiel 180

6-Hydroxymethyl-pyridin-4-carbonsäure-(glycyl-cyclopentyl)ester)amid

Beispiel 181

6-Hydroxymethyl-pyridin-4-carbonsäure-(glycyl-glycylmethylester)amid

Beispiel 182

6-Hydroxymethyl-pyridin-4-carbonsäure-(glycyl-glycyl-(2-propyl)ester)amid

Weitere Beispiele im Sinne der Erfindung, die analog hergestellt wurden, wobei Et = Ethyl, Me = Methyl, Bu = Butyl, Hex = Hexyl, Ph = Phenyl, C = Cyclo bedeuten.

1)

$R^3$ = 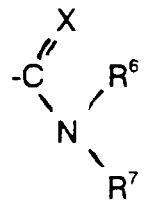

$Y = CH_2OR^5$ $X = O$

| n | $R^1$ | $R^2$ | $R^4$ | $R^5$ | $R^6$ | $R^7$ |
|---|---|---|---|---|---|---|
| 0 | H | H | H | Et | H | $CH_2CH_2OMe$ |
| 0 | H | H | H | Et | H | $CH_2CH_2OH$ |
| 0 | H | H | H | Et | H | $CH_2CH_2CH_2OMe$ |
| 0 | H | H | H | Et | H | $CH_2CH_2CH_2OH$ |
| 0 | H | H | H | Bu | H | $CH_2CH_2CH_2OMe$ |
| 0 | H | H | H | Bu | H | $CH_2CH_2CH_2OH$ |
| 0 | H | H | H | Ph | H | $CH_2CH_2OMe$ |
| 0 | H | H | H | Ph | H | $CH_2CH_2OH$ |
| 0 | H | Cl | H | Me | H | $CH_2CH_2OMe$ |
| 0 | H | Cl | H | H | H | $CH_2CH_2OMe$ |
| 0 | H | Cl | H | H | H | $CH_2CH_2OH$ |
| 0 | H | Me | H | H | H | $CH_2CH_2CH_2OMe$ |
| 0 | H | Me | H | H | H | $CH_2CH_2CH_2OH$ |
| 0 | H | H | H | CO-2-Me-Ph | H | $CH_2CH_2CH_2OMe$ |
| 0 | H | H | H | CO-2-Me-Ph | H | $CH_2CH_2CH_2OH$ |
| 0 | H | H | H | CO-c-Hex | H | $CH_2CH_2CH_2OMe$ |
| 0 | H | H | H | CO-c-Hex | H | $CH_2CH_2CH_2OH$ |
| 0 | H | H | H | H | H | $CH_2CH_2O$-CO-n-Bu |
| 0 | H | H | H | H | H | $CH_2CH_2O$-CO-Et |
| 0 | H | H | H | H | H | $CH_2CH_2O$-CO-c-Hex |
| 0 | H | H | H | H | H | $CH_2CH_2CH_2O$-CO-2-Me-Ph |
| 0 | H | H | H | H | H | $CH_2CH_2O$-CO-NHEt |

| n | $R^1$ | $R^2$ | $R^4$ | $R^5$ | $R^6$ | $R^7$ |
|---|---|---|---|---|---|---|
| 0 | H | H | H | H | H | $CH_2CH_2O$-CO-NHnBu |
| 0 | H | H | H | H | H | $CH_2CH_2O$-CO-NH-c-Hex |
| 0 | H | H | H | Me | H | $CH_2CH_2O$-CO-n-Bu |
| 0 | H | H | H | Me | H | $CH_2CH_2O$-CO-Ph |
| 0 | H | H | H | Me | H | $CH_2O$-CO-2-Me-Ph |
| 0 | H | H | H | H | H | $CH_2CH_2CH_2O$-CO-OEt |
| 0 | H | H | H | H | H | $CH_2CH_2CH_2O$-CO-O-c-Hex |

| n | $R^1$ | $R^2$ | $R^4$ | $R^5$ | $R^6$ | $R^7$ |
|---|---|---|---|---|---|---|
| 1 | H | H | H | Et | H | $CH_2CH_2OMe$ |
| 1 | H | H | H | Et | H | $CH_2CH_2OH$ |
| 1 | H | H | H | Et | H | $CH_2CH_2CH_2OMe$ |
| 1 | H | H | H | Et | H | $CH_2CH_2CH_2OH$ |
| 1 | H | H | H | Bu | H | $CH_2CH_2CH_2OMe$ |
| 1 | H | H | H | Bu | H | $CH_2CH_2CH_2OH$ |
| 1 | H | H | H | Ph | H | $CH_2CH_2OMe$ |
| 1 | H | H | H | Ph | H | $CH_2CH_2OH$ |
| 1 | H | Cl | H | Me | H | $CH_2CH_2OMe$ |
| 1 | H | Cl | H | H | H | $CH_2CH_2OMe$ |
| 1 | H | Cl | H | H | H | $CH_2CH_2OH$ |

| n | $R^1$ | $R^2$ | $R^4$ | $R^5$ | $R^6$ | $R^7$ |
|---|---|---|---|---|---|---|
| 1 | H | Me | H | H | H | $CH_2CH_2CH_2OMe$ |
| 1 | H | Me | H | H | H | $CH_2CH_2CH_2OH$ |
| 1 | H | H | H | CO-2-Me-Ph | H | $CH_2CH_2CH_2OMe$ |
| 1 | H | H | H | CO-2-Me-Ph | H | $CH_2CH_2CH_2OH$ |
| 1 | H | H | H | CO-c-Hex | H | $CH_2CH_2CH_2OMe$ |
| 1 | H | H | H | CO-c-Hex | H | $CH_2CH_2CH_2OH$ |
| 1 | H | H | H | H | H | $CH_2CH_2O$-CO-n-Bu |
| 1 | H | H | H | H | H | $CH_2CH_2O$-CO-Et |
| 1 | H | H | H | H | H | $CH_2CH_2O$-CO-c-Hex |
| 1 | H | H | H | H | H | $CH_2CH_2CH_2O$-CO-2-Me-Ph |
| 1 | H | H | H | H | H | $CH_2CH_2O$-CO-NHEt |
| 1 | H | H | H | H | H | $CH_2CH_2O$-CO-NH-n-Bu |
| 1 | H | H | H | H | H | $CH_2CH_2O$-CO-NH-c-Hex |
| 1 | H | H | H | Me | H | $CH_2CH_2O$-CO-n-Bu |
| 1 | H | H | H | Me | H | $CH_2CH_2O$-CO-Ph |
| 1 | H | H | H | Me | H | $CH_2O$-CO-2-Me-Ph |
| 1 | H | H | H | H | H | $CH_2CH_2CH_2O$-CO-OEt |
| 1 | H | H | H | H | H | $CH_2CH_2CH_2O$-CO-O-c-Hex |

$R^2$ = 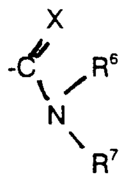

$Y = CH_2OR^5$ $X = O$

| n | $R^1$ | $R^3$ | $R^4$ | $R^5$ | $R^6$ | $R^7$ |
|---|---|---|---|---|---|---|
| 0 | H | H | H | Et | H | $CH_2CH_2OMe$ |
| 0 | H | H | H | Et | H | $CH_2CH_2OH$ |
| 0 | H | H | H | Et | H | $CH_2CH_2CH_2OMe$ |
| 0 | H | H | H | Et | H | $CH_2CH_2CH_2OH$ |
| 0 | H | H | H | Bn | H | $CH_2CH_2CH_2OMe$ |
| 0 | H | H | H | Bn | H | $CH_2CH_2CH_2OH$ |
| 0 | H | H | H | Ph | H | $CH_2CH_2OMe$ |
| 0 | H | H | H | Ph | H | $CH_2CH_2OH$ |
| 0 | H | Cl | H | Me | H | $CH_2CH_2OMe$ |
| 0 | H | Cl | H | H | H | $CH_2CH_2OMe$ |
| 0 | H | Cl | H | H | H | $CH_2CH_2OH$ |
| 0 | H | Me | H | H | H | $CH_2CH_2CH_2OMe$ |
| 0 | H | Me | H | H | H | $CH_2CH_2CH_2OH$ |
| 0 | H | H | H | CO-2-Me-Ph | H | $CH_2CH_2CH_2OMe$ |
| 0 | H | H | H | CO-2-Me-Ph | H | $CH_2CH_2CH_2OH$ |
| 0 | H | H | H | CO-c-Hex | H | $CH_2CH_2CH_2OMe$ |

| n | $R^1$ | $R^3$ | $R^4$ | $R^5$ | $R^6$ | $R^7$ |
|---|---|---|---|---|---|---|
| 0 | H | H | H | CO-c-Hex | H | $CH_2CH_2CH_2OH$ |
| 0 | H | H | H | H | H | $CH_2CH_2O$-CO-n-Bu |
| 0 | H | H | H | H | H | $CH_2CH_2O$-CO-Et |
| 0 | H | H | H | H | H | $CH_2CH_2O$-CO-c-Hex |
| 0 | H | H | H | H | H | $CH_2CH_2CH_2O$-CO-2-Me-Ph |
| 0 | H | H | H | H | H | $CH_2CH_2O$-CO-NHEt |
| 0 | H | H | H | H | H | $CH_2CH_2O$-CO-NH-n-Bu |
| 0 | H | H | H | H | H | $CH_2CH_2O$-CO-NH-c-Hex |
| 0 | H | H | H | Me | H | $CH_2CH_2O$-CO-n-Bu |
| 0 | H | H | H | Me | H | $CH_2CH_2O$-CO-Ph |
| 0 | H | H | H | Me | H | $CH_2O$-CO-2-Me-Ph |
| 0 | H | H | H | H | H | $CH_2CH_2CH_2O$-CO-OEt |
| 0 | H | H | H | H | H | $CH_2CH_2CH_2O$-CO-O-c-Hex |

| n | $R^1$ | $R^3$ | $R^4$ | $R^5$ | $R^6$ | $R^7$ |
|---|---|---|---|---|---|---|
| 1 | H | H | H | Et | H | $CH_2CH_2OMe$ |
| 1 | H | H | H | Et | H | $CH_2CH_2OH$ |
| 1 | H | H | H | Et | H | $CH_2CH_2CH_2OMe$ |
| 1 | H | H | H | Et | H | $CH_2CH_2CH_2OH$ |
| 1 | H | H | H | Bu | H | $CH_2CH_2CH_2OMe$ |

| n | $R^1$ | $R^3$ | $R^4$ | $R^5$ | $R^6$ | $R^7$ |
|---|---|---|---|---|---|---|
| 1 | H | H | H | Bu | H | $CH_2CH_2CH_2OH$ |
| 1 | H | H | H | Ph | H | $CH_2CH_2OMe$ |
| 1 | H | H | H | Ph | H | $CH_2CH_2OH$ |
| 1 | H | Cl | H | Me | H | $CH_2CH_2OMe$ |
| 1 | H | Cl | H | H | H | $CH_2CH_2OMe$ |
| 1 | H | Cl | H | H | H | $CH_2CH_2OH$ |
| 1 | H | Me | H | H | H | $CH_2CH_2CH_2OMe$ |
| 1 | H | Me | H | H | H | $CH_2CH_2CH_2OH$ |
| 1 | H | H | H | CO-2-Me-Ph | H | $CH_2CH_2CH_2OMe$ |
| 1 | H | H | H | CO-2-Me-Ph | H | $CH_2CH_2CH_2OH$ |
| 1 | H | H | H | CO-c-Hex | H | $CH_2CH_2CH_2OMe$ |
| 1 | H | H | H | CO-c-Hex | H | $CH_2CH_2CH_2OH$ |
| 1 | H | H | H | H | H | $CH_2CH_2O$-CO-n-Bu |
| 1 | H | H | H | H | H | $CH_2CH_2O$-CO-Et |
| 1 | H | H | H | H | H | $CH_2CH_2O$-CO-c-Hex |
| 1 | H | H | H | H | H | $CH_2CH_2CH_2O$-CO-2-Me-Ph |
| 1 | H | H | H | H | H | $CH_2CH_2O$-CO-NHEt |
| 1 | H | H | H | H | H | $CH_2CH_2O$-CO-NH-n-Bu |
| 1 | H | H | H | H | H | $CH_2CH_2O$-CO-NH-c-Hex |
| 1 | H | H | H | Me | H | $CH_2CH_2O$-CO-n-Bu |
| 1 | H | H | H | Me | H | $CH_2CH_2O$-CO-Ph |
| 1 | H | H | H | Me | H | $CH_2O$-CO-2-Me-Ph |

| n | $R^1$ | $R^3$ | $R^4$ | $R^5$ | $R^6$ | $R^7$ |
|---|---|---|---|---|---|---|
| 1 | H | H | H | H | H | $CH_2CH_2CH_2O$-CO-OEt |
| 1 | H | H | H | H | H | $CH_2CH_2CH_2O$-CO-O-c-Hex |

**Patentansprüche**

**1.** Pyridinderivate der allgemeinen Formel I

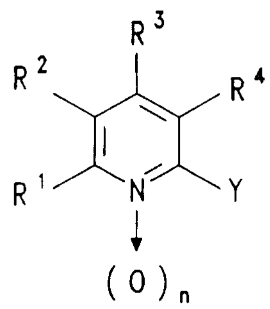

( I )

in der

$R^1$, $R^4$ und einer der beiden Reste $R^2$ oder $R^3$ gleich oder verschieden sind und Wasserstoff, Halogen, insbesondere Fluor, Chlor oder Brom, Cyano, Nitro, Trifluormethyl, ($C_1$-$C_{12}$)-Alkyl, Hydroxy, ($C_1$-$C_6$)-Hydroxyalkyl, ($C_1$-$C_{12}$)-Alkoxy, - O-$[CH_2]_xCH_{(2f+1-g)}F_g$,-$OCF_2Cl$, -O-$CF_2$-CHFCl, ($C_1$-$C_8$)-Alkylmercapto, ($C_1$-$C_8$)-Alkylsulfinyl, ($C_1$-$C_8$)-Alkylsulfonyl, ($C_1$-$C_8$)-Alkylcarbonyl, ($C_1$-$C_6$)-Alkoxycarbonyl, Carbamoyl, N-($C_1$-$C_4$)-Alkylcarbamoyl, N,N-Di-($C_1$-$C_4$)-alkylcarbamoyl, ($C_1$-$C_8$)-Alkylcarbonyloxy, ($C_3$-$C_8$)-Cycloalkyl, Phenyl, Benzyl, Phenoxy, Benzyloxy, Anilino, N-Methylanilino, Phenylmercapto, Phenylsulfonyl, Phenylsulfinyl, oder
-NR'-R", ($C_1$-$C_{12}$)-Alkoxy-($C_1$-$C_{12}$)-alkyl, ($C_1$-$C_{12}$)-Alkoxy-($C_1$-$C_{12}$)-alkoxy, bedeuten, oder einen unsubstituierten oder substituierten ($C_6$-$C_{12}$)-Aryloxyrest oder ($C_7$-$C_{11}$)-Aralkyloxyrest bedeutet, der 1,2,3,4 oder 5 gleiche oder verschiedene Substituenten aus der Reihe Halogen, Cyano, Nitro, Trifluormethyl, ($C_1$-$C_6$)-Alkyl, ($C_1$-$C_6$)-Hydroxyalkyl, ($C_1$-$C_6$)-Alkoxy, -O-$[CH_2-]_xCH_{2f+1-g}F_g$, -$OCF_2Cl$, -O-$CF_2$-CHFCl, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_4$)-Alkylcarbamoyl, N,N-Di-($C_1$-$C_4$)-alkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R", Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl, insbesondere bis zu 3 der vorstehend genannten gleichen oder verschiedenen Substituenten im Arylteil trägt, wobei eine $CH_2$-Gruppe der Alkylkette des Aralkyloxyrestes gegebenenfalls durch O, S, SO, $SO_2$ oder NR' ersetzt ist,

und der andere Rest, $R^2$ oder $R^3$, folgender allgemeiner Formel II entspricht

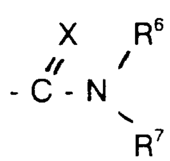

(II)

worin

X O oder S,

$R^6$ einen verzweigten oder unverzweigten ($C_1$-$C_{12}$)-Alkylrest, ($C_1$-$C_{12}$)-Alkenyl- oder ($C_1$-$C_{12}$)-Alkinylrest bedeutet, der einfach oder mehrfach substituiert ist mit

Halogen, Hydroxy, Cyano, Amino, Carboxyl, ($C_1$-$C_{10}$)-Alkoxy, ($C_1$-$C_{12}$)-Alkoxycarbonyl, ($C_3C_8$)-Cycloalkoxycarbonyl, ($C_7$-$C_{16}$)-Aralkoxycarbonyl, ($C_6$-$C_{12}$)-Aryloxycarbonyl, ($C_1$-$C_{12}$)-Alkylcarbonyloxy, ($C_1$-$C_{12}$)-Alkanoylamino, ($C_3$-$C_8$)-Cycloalkanoylamino, $C_1$-$C_{12}$)-Hydroxyalkanoylamino, ($C_6$-$C_{12}$)-Aroylamino, ($C_7$-$C_{11}$)-Aralkanoylamino, ($C_1$-$C_8$)-Alkoxy-($C_1$-$C_{12}$)-alkanoylamino, ($C_1$-$C_8$)-Alkoxycarbonyloxy, ($C_1$-$C_8$)-Alkoxy-($C_1$-$C_8$)-alkoxycarbonyloxy, ($C_6$-$C_{12}$)-Aryloxycarbonyloxy, ($C_7$-$C_{11}$)-Aralkyloxycarbonyloxy, ($C_7$-$C_{11}$)-Aralkylcarbonyloxy, ($C_6$-$C_{12}$)-Arylcarbonyloxy, ($C_3$-$C_8$)-Alkenylcarbonyloxy, ($C_3$-$C_8$)-Alkinylcarbonyloxy, ($C_3$-$C_8$)-Cycloalkylcarbonyloxy, ($C_1$-$C_{12}$)-Alkoxy-($C_1$-$C_{12}$)-alkoxy, ($C_1$-$C_{12}$)-Alkoxy-amino, ($C_1$-$C_{12}$)-Alkoxy-N-($C_1$-$C_6$)-alkylamino, ($C_1$-$C_{12}$)-Alkoxy-N,N-($C_1$-$C_6$)-dialkylamino, Carbamoyloxy, N-($C_1$-$C_8$)-Alkylcarbamoyloxy, N,N-Di-($C_1$-$C_8$)-alkylcarbamoyloxy, N-($C_3$-$C_8$)-Cycloalkylcarbamoyloxy, ($C_1$-$C_8$)-Alkylmercapto, ($C_1$-$C_8$)-Alkylsulfinyl, ($C_1$-$C_8$)-Alkylsulfonyl, ($C_1$-$C_8$)-Alkylcarbonyl, ($C_3$-$C_8$)-Cycloalkylcarbonyl, Nitro, Trifluormethyl, Phenylmercapto, Phenylsulfonyl, Phenylsulfinyl, Sulfamoyl, N-($C_1$-$C_6$)-Alkylsulfamoyl, N,N-Di-($C_1$-$C_6$)-alkylsulfamoyl, ($C_1$-$C_8$)-Alkylsulfonamido, Arylsulfonamido, Carbamoyl, N-($C_1$-$C_8$)-Alkylcarbamoyl,N,N-Di-($C_1$-$C_8$)-alkylcarbamoyl, N-($C_3$-$C_8$)-Cycloalkylcarbamoyl, N-($C_6$-$C_{12}$)-Arylcarbamoyl, N-($C_7$-$C_{16}$)-Aralkylcarbamoyl, N-($C_1$-$C_{10}$)-Alkyl-N-($C_6$-$C_{12}$)-arylcarbamoyl, N-($C_1$-$C_{10}$)-Alkyl-N-($C_7$-$C_{16}$)-aralkylcarbamoyl, N-(($C_1$-$C_8$)-Alkoxy-($C_1$-$C_4$)alkyl)carbamoyl, N-(($C_1$-$C_4$)-Hydroxyalkyl)carbamoyl oder ein O-acyliertes Derivat hiervon oder mit

einem substituierten ($C_6$-$C_{12}$)-Arylrest, einem ($C_7$-$C_{11}$)Aralkylrest oder einem Heteroarylrest, der 1,2,3,4 oder 5 gleiche oder verschiedene Substituenten aus der Reihe Hydroxy, Halogen, Cyano, Nitro, ($C_1$-$C_6$)-Alkyl, ($C_1$-$C_6$)-Alkoxy, Trifluormethyl, ($C_1$-$C_6$)-Hydroxyalkyl, $-O-[CH_2-]_xC_fH_{(2f+1-g)}F_g$, $-OCF_2Cl$, $-OCF_2-CHFCl$, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_8$)-Alkylcarbamoyl, N,N-Di-($C_1$-$C_8$)-alkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl insbesondere bis zu 3 der vorstehend genannten gleichen oder verschiedenen Substituenten im Arylteil trägt, oder mit

einem substituierten ($C_6$-$C_{12}$)-Aryloxyrest, ($C_7$-$C_{11}$)Aralkyloxyrest oder einem Heteroaryloxyrest, der 1,2,3,4 oder 5 gleiche oder verschiedene Substituenten aus der Reihe Hydroxy, Halogen, Cyano, Nitro, ($C_1$-$C_6$)-Alkyl, ($C_1$-$C_6$)-Alkoxy, Trifluormethyl, ($C_1$-$C_6$)-Hydroxyalkyl, $-O-[CH_2-]_xC_fH_{(2f+1-g)}F_g$, $- OCF_2Cl$, $-OCF_2-CHFCl$, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_4$)-Alkylcarbamoyl, N,N-Di-($C_1$-$C_4$)-alkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl insbesondere bis zu 3 der vorstehend genannten gleichen oder verschiedenen Substituenten im Arylteil trägt,

$R^6$ einen ($C_1$-$C_{12}$)-Alkoxy-, ($C_3$-$C_8$)-Cycloalkoxyrest oder einen ($C_6$-$C_{12}$)-Arylrest, ($C_7$-$C_{11}$)-Aralkylrest oder einen Heteroarylrest bedeutet, der 1,2,3,4 oder 5 gleiche oder verschiedene Substituenten aus der Reihe Hydroxy, Halogen, Cyano, Nitro, ($C_1$-$C_6$)-Alkyl, ($C_1$-$C_6$)-Alkoxy, Trifluormethyl, ($C_1$-$C_6$)-Hydroxyalkyl, $-O-[CH_2-]_xC_fH_{(2f+1-g)}F_g$, $-OCF_2Cl$, $-OCF_2-CHFCl$, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_8$)-Alkylcarbamoyl, N,N-Di-($C_1$-$C_8$)-alkylcarbamoyl, N-($C_3$-$C_8$)Cycloalkylcarbamoyl, N-($C_6$-$C_{12}$)-Arylcarbamoyl, N-($C_7$-$C_{16}$)-Aralkylcarbamoyl, N-($C_1$-$C_{10}$)-Alkyl-N-($C_6$-$C_{12}$)arylcarbamoyl, N-($C_1$-$C_{10}$)-Alkyl-N-($C_7$-$C_{16}$)aralkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl trägt, insbesondere bis zu 3 der vorstehend genannten gleichen oder verschiedenen Substituenten, im Arylteil trägt,

worin jeweils

R' und R'' gleich oder verschieden sind und Wasserstoff, ($C_6$-$C_{12}$)-Aryl, ($C_1$-$C_8$)-Alkyl, ($C_1$-$C_8$)-Aminoalkyl, N-($C_1$-$C_8$)-Alkylamino-($C_1$-$C_{12}$)-alkyl, N,N-Di-($C_1$-$C_8$)-alkylamino-($C_1$-$C_{12}$)-al-

kyl, $(C_7-C_{14})$-Aralkyl bedeuten oder gemeinsam für - $[CH_2]_n$- stehen, worin eine $CH_2$-Gruppe durch O,S,N-$(C_1-C_4)$-Alkanoylimino oder N-$(C_1-C_4)$-Alkoxycarbonylamino ersetzt sein kann,

und

$R^6$ substituiert sein kann mit einem Rest der allgemeinen Formel III

- O - Z, (III)

worin

Z eine über ihren Acylrest gebundene Aminosäure oder eines Derivates hiervon oder $R^5$ bedeutet, oder mit einem Rest der allgemeinen Formel IV

- CO - U (IV)

worin

U ein über die Aminogruppe gebundenes Polypeptid, vorzugsweise eine Tri-oder ein Dipeptid oder ein über die Aminogruppe gebundenes Aminosäurederivat, vorzugsweise ein L-Aminosäure-Derivat, insbesondere ein L-Alanin- oder ein Glycinderivat, bedeutet,

$R^7$ unabhängig von $R^6$ Wasserstoff oder $R^6$ sein kann, wobei die Reste $R^6$ und $R^7$ auch zusammen mit dem Stickstoffatom einen 5-, 6- oder 7-gliedrigen gesättigten heterocyclischen Ring bilden, der heterocyclische Ring auch ein zweites Stickstoffatom beinhalten kann und der heterocyclische Ring seinerseits mit Phenyl oder Phenyl-$C_1$-$C_3$-Alkyl substituiert sein kann,

Y Methyl oder $CH_2OR^5$ bedeutet, wobei

$R^5$ Wasserstoff, Z oder den Rest einer physiologisch verträglichen, vorzugsweise in der Leber oder unter physiologischen Bedingungen abspaltbaren Alkohol-Schutzgruppe bedeutet, und

n 0 der 1

f 1 bis 8, vorzugsweise 1 bis 5

g 0,1 bis (2f + 1) sowie

x 0, 1, 2 oder 3, vorzugsweise 0 oder 1 ist, zuzüglich alle Derivate, die in der entsprechenden Amino- oder Hydroxygruppe eine Schutzgruppe aufweisen sowie die physiologisch wirksamen Salze.

**2.** Verbindungen nach Anspruch 1, in denen

$R^1$, $R^4$ und einer der beiden Reste $R^2$ oder $R^3$ gleich oder verschieden sind und Wasserstoff, Halogen, insbesondere Fluor, Chlor oder Brom, $(C_1-C_5)$-Alkyl, Hydroxy, $(C_1-C_5)$-Alkoxy, $(C_1-C_5)$-Alkoxycarbonyl,

$(C_1-C_8)$-Alkylcarbonyloxy, -NR'-R'', $(C_1-C_4)$-Alkoxy-$(C_1-C_6)$-alkyl, $(C_1-C_6)$Alkoxy-$(C_1-C_6)$-alkoxy oder einen substituierten $(C_6-C_{12})$-Aryloxy- oder $(C_7-C_{11})$-Aralkyloxyrest bedeuten, der 1, 2 oder 3 gleiche oder verschiedene Substituenten aus der Reihe, Halogen, insbesondere, Fluor oder Chlor Cyano, Trifluormethyl, $(C_1-C_5)$-Alkyl, $(C_1-C_6)$-Alkoxy, $(C_1-C_5)$-Alkylcarbonyl, $(C_1-C_5)$-Alkoxycarbonyl, trägt, wobei

R' und R'' gleich oder verschieden sind und Wasserstoff, $(C_6-C_{12})$-Aryl, $(C_1-C_8)$-Alkyl oder $(C_1-C_6)$-Alkylcarbonyl bedeutet,

n 0 oder 1 ist und

der andere Rest $R^2$ oder $R^3$ der allgemeinen Formel II entspricht, worin

X O,

Y $CH_2OR^5$,

$R^6$ einen verzweigten oder unverzweigten $(C_1-C_8)$-Alkylrest der einfach oder zweifach, insbesondere einfach substituiert ist mit

Hydroxy, $(C_1-C_{10})$-Alkoxy, $(C_1-C_8)$-Alkoxycarbonyl,$(C_3-C_8)$-Cycloalkoxycarbonyl,$(C_7-C_{12})$-Aralkoxycarbonyl, $(C_6-C_{10})$-Aryloxycarbonyl, Carbamoyl, N-($(C_1-C_8)$-Alkoxy-$(C_1-C_2)$alkyl)-carbamoyl, N-($(C_1-C_4)$-Hydroxyalkyl)carbamoyl oder ein O-acyliertes Derivat hiervon, $(C_1-C_{12})$-Alkylcarbonyloxy, Carboxyl, $(C_1-C_8)$-Alkoxycarbonyloxy, $(C_6-C_{12})$-Aryloxycarbonyloxy, -$(C_7-C_{11})$-Aralkyloxycarbonyloxy, $(C_7-C_{11})$-Aralkylcarbonyloxy, $(C_6-C_{12})$-Arylcarbonyloxy, $(C_3-C_8)$-Cycloalkylcarbonyloxy, Carbamoyloxy, N-$(C_1-C_6)$-Alkylcarbamoyloxy, N,N-Di-$(C_1-C_8)$-alkylcarbamoyloxy, N-$(C_3-C_8)$-Cycloalkylcarbamoyloxy, $(C_1-C_8)$-Alkylcarbonyl, $(C_3-C_8)$-Cycloalkylcarbonyl, NR'R'', oder dem Acylrest einer N-derivatisierten Aminosäure, oder mit

einem substituierten ($C_6$-$C_{12}$)Arylrest, der ein oder zwei Substituenten aus der Reihe Halogen, insbesondere Fluor, Chlor, Trifluormethyl, Hydroxy, ($C_1$-$C_4$)-Alkoxy, ($C_1$-$C_5$)-Alkyl, ($C_1$-$C_6$)-Alkylcarbonyloxy, ($C_1$-$C_6$)-Alkoxycarbonyl, ($C_1$-$C_4$)-Hydroxyalkyl aufweist, oder mit

einem substituierten ($C_6$-$C_{12}$)-Aryloxyrest, ($C_7$-$C_{11}$)-Aralkyloxyrest oder einem Heteroaryloxyrest, insbesondere einem Phenoxyrest oder einem Benzyloxyrest, der ein oder zwei Substituenten aus der Reihe Hydroxy, ($C_1$-$C_4$)-Alkoxy, ($C_1$-$C_5$)-Alkyl, ($C_1$-$C_6$)-Alkylcarbonyloxy, ($C_1$-$C_6$)-Alkoxycarbonyl, ($C_1$-$C_4$)-Hydroxyalkyl aufweist,

$R^6$ einen ($C_6$-$C_{12}$)-Arylrest, der 1, 2 oder 3-fach substituiert ist mit Hydroxy, Halogen, insbesondere Chlor oder Brom, Cyano, Nitro, ($C_1$-$C_6$)-Alkyl, ($C_1$-$C_6$)-Alkoxy, Trifluormethyl, ($C_1$-$C_6$)-Hydroxyalkyl, $-O-[CH_2]_xC_fH_{(2f+1g)}F_g$. - $OCF_2Cl$, $OCF_2$-CHFCl, ($C_1$-$C_6$)-Alkylcarbonyl, ($C_1$-$C_6$)-Alkoxycarbonyl, Carbamoyl, N-($C_1$-$C_8$)-Alkylcarbamoyl, N,N-Di-($C_1$-$C_8$)-alkylcarbamoyl, ($C_1$-$C_6$)-Alkylcarbonyloxy, Phenyl, Benzyl, Phenoxy, Benzyloxy und/oder NR'R",

$R^7$ unabhängig von $R^6$ Wasserstoff oder ($C_1$-$C_5$)Alkyl sein kann,

$R^5$ H oder eine Alkohol-Schutzgruppe sein kann, wobei $OR^5$ insbesondere einen substituierten oder unsubstituierten Methylether, einen substituierten oder unsubstituierten Ethylether, einen substituierten oder unsubstituierten Benzylether, einen Silylether, einen Ester, ein Carbonat, ein Carbamat oder ein Sulfonat bedeutet.

**3.** Verbindungen den Ansprüchen 1 oder 2, in denen $R^5$ Wasserstoff, ($C_1$-$C_8$)-Alkyl, Benzyl, ($C_1$-$C_8$)-Alkylcarbonyl, Phenylcarbonyl und Carbamoyl sind.

**4.** Verbindungen nach den Ansprüchen 1 bis 3, in denen n = 0 ist und Y = $CH_2OR^5$,

$R^1$ und $R^4$ sowie einer der Reste $R^2$ oder $R^3$, insbesondere $R^2$, Wasserstoff, ($C_1$-$C_5$)-Alkyl, Fluor, Chlor, ($C_1$-$C_5$)-Alkoxy,

und der andere Rest, $R^2$ oder $R^3$, insbesondere $R^3$ der allgemeinen Formel II entspricht, worin

x O,

$R^6$ verzweigtes oder unverzweigtes ($C_1$-$C_3$)-Alkyl, welches einfach substituiert ist mit Hydroxy, ($C_1$-$C_4$)-Alkoxy. ($C_1$-$C_9$)-Alkoxycarbonyl, ($C_3$-$C_6$)-Cycloalkoxycarbonyl, Benzyloxycarbonyl, ($C_1$-$C_4$)-Alkylcarbonyloxy und/oder (($C_1$-$C_9$)-Alkoxycarbonyl)methyl)-carbamoyl, oder

$R^6$ ($C_6$-$C_{12}$)Aryl, welches einfach oder zweifach substituiert ist mit Hydroxy, Halogen, ($C_1$-$C_6$)-Alkoxy, ($C_1$-$C_6$)-Alkyl, Trifluormethyl, ($C_1$-$C_6$)-Hydroxyalkyl, $-O-[CH_2]-_xC_fH_{(2f+1-g)}F_g$ und/oder NR'R", bedeutet und

$R^7$ Wasserstoff ist.

**5.** Verbindungen nach den Ansprüchen 1 bis 4, in denen $R^6$ jeweils endständig substituiert ist.

**6.** Verbindungen nach den Ansprüchen 1 bis 5, in denen $R^1$, $R^4$ sowie einer der beiden Reste $R^2$ oder $R^3$ Wasserstoff bedeuten.

**7.** Verfahren zur Herstellung von Verbindungen gemäß den Ansprüchen 1 bis 6, dadurch gekennzeichnet, daß Pyridin-4- oder -5-carbonsäuren oder ihre Ester gemäß Formel VI

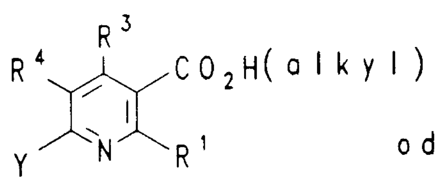

oder

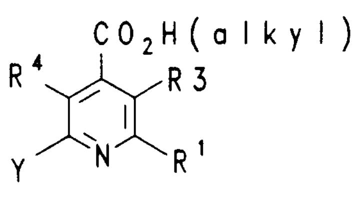

VI

mit einem Amin

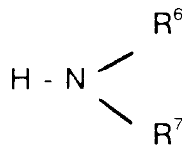

zu Verbindungen der Formel I umgesetzt werden.

**8.** Verfahren zur Herstellung von Verbindungen gemäß den Ansprüchen 1 bis 6, in denen Y = $-CH_2-OR^5$ bedeutet und gemäß Anspruch 7, dadurch gekennzeichnet, daß Pyridin-4- oder -5-carbonsäuren der Formel VII, in denen Y' ein Substituent bedeutet, der sich in einen Rest $-CH_2-OR^5$ umwandeln läßt,

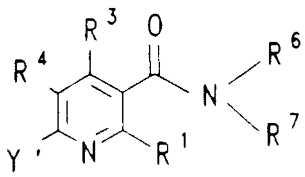

oder

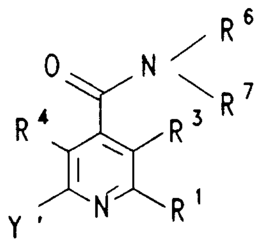

VII

a) sofern Y' = $CH_3$ und n = 0 bedeuten, zu Verbindungen der Formel VII halogeniert werden, in denen Y' = $CH_2W$ bedeutet, worin W für Halogen, insbesondere Chlor oder Brom, steht und diese Verbindungen ihrerseits mit Reagenzien der Formel $MOR^5$ zu Verbindungen der Formel I umgesetzt werden, worin M Wasserstoff, ein Alkali- oder ein Erdalkaliatom bedeutet, oder b) sofern Y' = $CH_3$ und n = 1 bedeuten, mit $Ac_2O/AcOH$ zu Verbindungen der Formel I umgesetzt werden, in denen Y = $-CH_2-OAc$ bedeutet, oder

c) sofern Y' = $-CO_2H$, $-CO_2Alkyl$ oder -CHO und n = 0 bedeuten, zu Verbindungen der Formel I, in denen Y = $CH_2OH$ bedeutet, reduziert werden.

**9.** Verbindungen nach einem der Ansprüche 1 bis 6 zur Anwendung als Fibrosuppressiva.

**10.** Verbindungen nach den Ansprüchen 1 bis 6 zur Beeinflussung des Stoffwechsels von Kollagen und kollagenähnlichen Stoffen.

**11.** Arzneimittel, enthaltend eine Verbindung der Formel I und einen verträglichen pharmazeutischen Träger.

**12.** Verwendung von Verbindungen der Formel I zur Beeinflussung des Stoffwechsels von Kollagen und kollagenähnlichen Stoffen.

**13.** Verwendung von Verbindungen der Formel I als Fibrosuppressiva.

**14.** Verfahren zur Herstellung von Arzneimitteln zur Beeinflussung des Stoffwechsels von Kollagen und kollagenähnlichen Stoffen, dadurch gekennzeichnet, daß das Arzneimittel eine Verbindung der Formel I enthält.

**Patentansprüche für folgende Vertragsstaaten : ES, GR**

**1.** Verfahren zur Herstellung von Pyridinderivate der allgemeinen Formel I

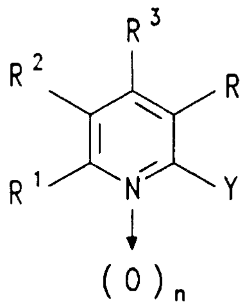

(I)

in denen

$R^1$, $R^4$ und einer der beiden Reste $R^2$ oder $R^3$ gleich oder verschieden sind und Wasserstoff, Halogen, insbesondere Fluor, Chlor oder Brom, Cyano, Nitro, Trifluormethyl, $(C_1\text{-}C_{12})$-Alkyl, Hydroxy, $(C_1\text{-}C_6)$-Hydroxyalkyl, $(C_1\text{-}C_{12})$-Alkoxy, - $O\text{-}[CH_2\text{-}]_xC_fH_{(2f+1\text{-}g)}F_g$, $\text{-}OCF_2Cl$, $\text{-}O\text{-}CF_2\text{-}CHFCl$, $(C_1\text{-}C_8)$-Alkylmercapto, $(C_1\text{-}C_8)$-Alkylsulfinyl, $(C_1\text{-}C_8)$-Alkylsulfonyl, $(C_1\text{-}C_8)$-Alkylcarbonyl, $(C_1\text{-}C_6)$-Alkoxycarbonyl, Carbamoyl, N-$(C_1\text{-}C_4)$-Alkylcarbamoyl, N,N-Di-$(C_1\text{-}C_4)$-alkylcarbamoyl, $(C_1\text{-}C_8)$-Alkylcarbonyloxy, $(C_3\text{-}C_8)$-Cycloalkyl, Phenyl, Benzyl, Phenoxy, Benzyloxy., Anilino, N-Methylanilino, Phenylmercapto, Phenylsulfonyl, Phenylsulfinyl, oder

-NR'-R'', $(C_1\text{-}C_{12})$-Alkoxy-$(C_1\text{-}C_{12})$-alkyl, $(C_1\text{-}C_{12})$-Alkoxy-$(C_1\text{-}C_{12})$-alkoxy, bedeuten, oder einen unsubstituierten oder substituierten $(C_6\text{-}C_{12})$-Aryloxyrest oder

$(C_7\text{-}C_{11})$-Aralkyloxyrest bedeutet, der 1,2,3,4 oder 5 gleiche oder

verschiedene Substituenten aus der Reihe Halogen, Cyano, Nitro, Trifluormethyl, $(C_1\text{-}C_6)$-Alkyl, $(C_1\text{-}C_6)$-Hydroxyalkyl, $(C_1\text{-}C_6)$-Alkoxy, $\text{-}O\text{-}[CH_2\text{-}]_xC_fH_{2f+1\text{-}g)}F_g$, $\text{-}OCF_2Cl$, $\text{-}O\text{-}CF_2\text{-}CHFCl$, $(C_1\text{-}C_6)$-Alkylmercapto, $(C_1\text{-}C_6)$-Alkylsulfinyl, $(C_1\text{-}C_6)$-Alkylsulfonyl, $(C_1\text{-}C_6)$-Alkylcarbonyl, $(C_1\text{-}C_6)$-Alkoxycarbonyl, Carbamoyl, N-$(C_1\text{-}C_4)$-Alkylcarbamoyl, N,N-Di-$(C_1\text{-}C_4)$-alkylcarbamoyl, $(C_1\text{-}C_6)$-Alkylcarbonyloxy, $(C_3\text{-}C_8)$-Cycloalkyl, Phenyl, Benzyl, Phenoxy, Benzyloxy, NR'-R'', Phenylmercapto, Phenylsulfonyl, Phenylsulfinyl, Sulfamoyl, N-$(C_1\text{-}C_4)$-Alkylsulfamoyl oder N,N-Di-$(C_1\text{-}C_4)$-alkylsulfamoyl, insbesondere bis zu 3 der vorstehend genannten gleichen oder verschiedenen Substituenten im Arylteil trägt, wobei eine $CH_2$-Gruppe der Alkylkette des Aralkyloxyrestes gegebenenfalls durch O, S, SO, $SO_2$ oder NR' ersetzt ist,

und der andere Rest, $R^2$ oder $R^3$, folgender allgemeiner Formel II entspricht

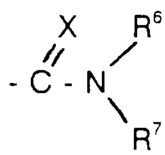

(II)

worin

X O oder S,

$R^6$ einen verzweigten oder unverzweigten $(C_1\text{-}C_{12})$-Alkylrest, $(C_1\text{-}C_{12})$-Alkenyl- oder $(C_1\text{-}C_{12})$-Alkinylrest bedeutet, der einfach oder mehrfach substituiert ist mit

Halogen, Hydroxy, Cyano, Amino, Carboxyl, $(C_1\text{-}C_{10})$-Alkoxy, $(C_1\text{-}C_{12})$-Alkoxycarbonyl, $(C_3\text{-}C_8)$-Cycloalkoxycarbonyl, $(C_7\text{-}C_{16})$-Aralkoxycarbonyl, $(C_6\text{-}C_{12})$-Aryloxycarbonyl, $(C_1\text{-}C_{12})$-Alkylcarbonyloxy, $(C_1\text{-}C_{12})$-Alkanoylamino, $(C_3\text{-}C_8)$-Cycloalkanoylamino, $C_1\text{-}C_{12})$-Hydroxyalkanoylamino, $(C_6\text{-}C_{12})$-Aroylamino, $(C_7\text{-}C_{11})$-Aralkanoylamino, $(C_1\text{-}C_8)$-Alkoxy-$(C_1\text{-}C_{12})$-alkanoylamino, $(C_1\text{-}C_8)$-Alkoxycarbonyloxy, $(C_1\text{-}C_8)$-Alkoxy-$(C_1\text{-}C_8)$-alkoxycarbonyloxy, $(C_6\text{-}C_{12})$-Aryloxycarbonyloxy, $(C_7\text{-}C_{11})$-Aralkyloxycarbonyloxy, $(C_7\text{-}C_{11})$-Aralkylcarbonyloxy, $(C_6\text{-}C_{12})$-Arylcarbonyloxy, $(C_3\text{-}C_8)$-Alkenylcarbonyloxy, $(C_3\text{-}C_8)$-Alkinylcarbonyloxy, $(C_3\text{-}C_8)$-Cycloalkylcarbonyloxy, $(C_1\text{-}C_{12})$-Alkoxy-$(C_1\text{-}C_{12})$-alkoxy, $(C_1\text{-}C_{12})$-Alkoxy-amino, $(C_1\text{-}C_{12})$-Alkoxy-N-$(C_1\text{-}C_6)$-alkylamino, $(C_1\text{-}C_{12})$-Alkoxy-N,N-$(C_1\text{-}C_6)$-dialkylamino, Carbamoyloxy, N-$(C_1\text{-}C_8)$-Alkylcarbamoyloxy, N,N-Di-$(C_1\text{-}C_8)$-alkylcarbamoyloxy, N-$(C_3\text{-}C_8)$-Cycloalkylcarbamoyloxy, $(C_1\text{-}C_8)$-Alkylmercapto, $(C_1\text{-}C_8)$-Alkylsulfinyl, $(C_1\text{-}C_8)$-Alkylsulfonyl, $(C_1\text{-}C_8)$-Alkylcarbonyl, $(C_3\text{-}C_8)$-Cycloalkylcarbonyl, Nitro, Trifluormethyl, Phenylmercapto, Phenylsulfonyl, Phenylsulfinyl, Sulfa-

moyl, N-($C_1$-$C_6$)-Alkylsulfamoyl, N,N-Di-($C_1$-$C_6$)-alkylsulfamoyl, ($C_1$-$C_8$)-Alkylsulfonamido, Arylsulfonamido, Carbamoyl, N-($C_1$-$C_8$)-Alkylcarbamoyl, N,N-Di-($C_1$-$C_8$)-alkylcarbamoyl, N-($C_3$-$C_8$)-Cycloalkylcarbamoyl, N-($C_6$-$C_{12}$)-Arylcarbamoyl, N-($C_7$-$C_{16}$)-Aralkylcarbamoyl, N-($C_1$-$C_{10}$)-Alkyl-N-($C_6$-$C_{12}$)-arylcarbamoyl, N-($C_1$-$C_{10}$)-Alkyl-N-($C_7$-$C_{16}$)aralkylcarbamoyl, N-(($C_1$-$C_8$)-Alkoxy-($C_1$-$C_4$)alkyl)carbamoyl, N-(($C_1$-$C_4$)-Hydroxyalkyl)carbamoyl oder ein O-acyliertes Derivat hiervon oder mit

einem substituierten ($C_6$-$C_{12}$)-Arylrest, einem ($C_7$-$C_{11}$)Aralkylrest oder einem Heteroarylrest, der 1,2,3,4 oder 5 gleiche oder verschiedene Substituenten aus der Reihe Hydroxy, Halogen, Cyano, Nitro, ($C_1$-$C_6$)-Alkyl, ($C_1$-$C_6$)-Alkoxy, Trifluormethyl, ($C_1$-$C_6$)-Hydroxyalkyl, $-O-[CH_2-]_xC_fH_{(2f+1-g)}F_g$, $-OCF_2Cl$, $-OCF_2-CHFCl$, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_8$)-Alkylcarbamoyl, N,N-Di-($C_1$-$C_8$)-alkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl insbesondere bis zu 3 der vorstehend genannten gleichen oder verschiedenen Substituenten im Arylteil trägt, oder mit

einem substituierten ($C_6$-$C_{12}$)-Aryloxyrest, ($C_7$-$C_{11}$)Aralkyloxyrest oder einem Heteroaryloxyrest, der 1,2,3,4 oder 5 gleiche oder verschiedene Substituenten aus der Reihe Hydroxy, Halogen, Cyano, Nitro, ($C_1$-$C_6$)-Alkyl, ($C_1$-$C_6$)-Alkoxy, Trifluormethyl, ($C_1$-$C_6$)-Hydroxyalkyl, $-O-[CH_2-]_xC_fH_{(2f+1+g)}F_g$, - $OCF_2Cl$, $-OCF_2-CHFCl$, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_4$)-Alkylcarbamoyl, N,N-Di-($C_1$-$C_4$)-alkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl insbesondere bis zu 3 der

vorstehend genannten gleichen oder verschiedenen Substituenten im Arylteil trägt,

$R^6$ einen ($C_1$-$C_{12}$)-Alkoxy-, ($C_3$-$C_8$)-Cycloalkoxyrest oder einen ($C_6$-$C_{12}$)-Arylrest, ($C_7$-$C_{11}$)-Aralkylrest oder einen Heteroarylrest bedeutet, der 1,2,3,4 oder 5 gleiche oder verschiedene Substituenten aus der Reihe Hydroxy, Halogen, Cyano, Nitro, ($C_1$-$C_6$)-Alkyl, ($C_1$-$C_6$)-Alkoxy, Trifluormethyl, ($C_1$-$C_6$)-Hydroxyalkyl, $-O-[CH_2-]_xC_fH_{(2f+1-g)}F_g$, $-OCF_2Cl$, $-OCF_2-CHFCl$, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_8$)-Alkylcarbamoyl, N,N-Di-($C_1$-$C_8$)-alkylcarbamoyl, N-($C_3$-$C_8$)Cycloalkylcarbamoyl, N-($C_6$-$C_{12}$)-Arylcarbamoyl, N-($C_7$-$C_{16}$)-Aralkylcarbamoyl, N-($C_1$-$C_{10}$)-Alkyl-N-($C_6$-$C_{12}$)arylcarbamoyl, N-($C_1$-$C_{10}$)-Alkyl-N-($C_7$-$C_{16}$)aralkylcarbamoyl, ($C_1$-$C_6$)-Alkylcarbonyloxy, ($C_3$-$C_8$)-Cycloalkyl, Phenyl, Benzyl, Phenoxy, Benzyloxy, NR'-R'', Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl trägt, insbesondere bis zu 3 der vorstehend genannten gleichen oder verschiedenen Substituenten, im Arylteil trägt,

worin jeweils
R' und R'' gleich oder verschieden sind und Wasserstoff, ($C_6$-$C_{12}$)-Aryl, ($C_1$-$C_8$)-Alkyl, ($C_1$-$C_8$)-Aminoalkyl, N-($C_1$-$C_8$)-Alkylamino-($C_1$-$C_{12}$)-alkyl, N,N-Di-($C_1$-$C_8$)-alkylamino-($C_1$-$C_{12}$)-alkyl, ($C_7$-$C_{14}$)-Aralkyl bedeuten oder gemeinsam für - $[CH_2]_n$- stehen, worin eine $CH_2$-Gruppe durch O,S,N-($C_1$-$C_4$)-Alkanoylimino oder N-($C_1$-$C_4$)-Alkoxycarbonylamino ersetzt sein kann,

und
$R^6$ substituiert sein kann mit einem Rest der allgemeinen Formel III

- O - Z, (III)

worin
Z eine über ihren Acylrest gebundene Aminosäure oder eines Derivates hiervon oder $R^5$ bedeutet, oder mit einem Rest der allgemeinen Formel IV

- CO - U (IV)

worin

U ein über die Aminogruppe gebundenes Polypeptid, vorzugsweise eine Tri-oder ein Dipeptid oder ein über die Aminogruppe gebundenes Aminosäurederivat, vorzugsweise ein L-Aminosäure-Derivat, insbesondere ein L-Alanin- oder ein Glycinderivat, bedeutet,

$R^7$ unabhängig von $R^6$ Wasserstoff oder $R^6$ sein kann, wobei die Reste $R^6$ und $R^7$ auch zusammen mit dem Stickstoffatom einen 5-, 6- oder 7-gliedrigen gesättigten heterocyclischen Ring bilden, der heterocyclische Ring auch ein zweites Stickstoffatom beinhalten kann und der heterocyclische Ring seinerseits mit Phenyl oder Phenyl-$C_1$-$C_3$-Alkyl substituiert sein kann,

Y Methyl oder $CH_2OR^5$ bedeutet, wobei

$R^5$ Wasserstoff, Z oder den Rest einer physiologisch verträglichen, vorzugsweise in der Leber oder unter physiologischen Bedingungen abspaltbaren Alkohol-Schutzgruppe bedeutet, und

n 0 der 1

f 1 bis 8, vorzugsweise 1 bis 5

g 0,1 bis (2f + 1) sowie

x 0, 1, 2 oder 3, vorzugsweise 0 oder 1 ist,

zuzüglich alle Derivate, die in der entsprechenden Amino- oder Hydroxygruppe eine Schutzgruppe aufweisen sowie die physiologisch wirksamen Salze, in dem man Pyridin-4- oder -5-carbonsäuren oder ihre Ester gemäß Formel VI

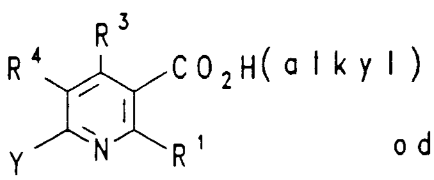

oder

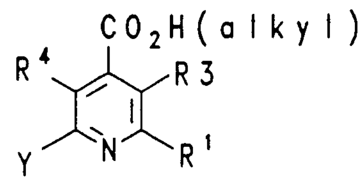

V I

mit einem Amin

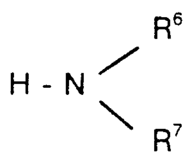

zu Verbindungen der Formel I umsetzt.

**2.** Verfahren nach Anspruch 1, in denen

$R^1$, $R^4$ und einer der beiden Reste $R^2$ oder $R^3$ gleich oder verschieden sind und Wasserstoff, Halogen, insbesondere Fluor, Chlor oder Brom, ($C_1$-$C_5$)-Alkyl, Hydroxy, ($C_1$-$C_5$)-Alkoxy, ($C_1$-$C_5$)-Alkoxycarbonyl,
($C_1$-$C_8$)-Alkylcarbonyloxy, -NR'-R'', ($C_1$-$C_4$)-Alkoxy-($C_1$-$C_6$)-alkyl, ($C_1$-$C_6$)Alkoxy-($C_1$-$C_6$)-alkoxy oder einen substituierten ($C_6$-$C_{12}$)-Aryloxy- oder ($C_7$-$C_{11}$)-Aralkyloxyrest bedeuten, der 1, 2 oder 3 gleiche oder verschiedene Substituenten aus der Reihe, Halogen, insbesondere, Fluor oder Chlor Cyano, Trifluormethyl, ($C_1$-$C_5$)-Alkyl, ($C_1$-$C_6$)-Alkoxy, ($C_1$-$C_5$)-Alkylcarbonyl, ($C_1$-$C_5$)-Alkoxycarbonyl, trägt, wobei

R' und R'' gleich oder verschieden sind und Wasserstoff, ($C_6$-$C_{12}$)-Aryl, ($C_1$-$C_8$)-Alkyl oder ($C_1$-$C_6$)-Alkylcarbonyl bedeutet,

n 0 oder 1 ist und

der andere Rest $R^2$ oder $R^3$ der allgemeinen Formel II entspricht, worin

X O,

Y $CH_2OR^5$,

$R^6$ einen verzweigten oder unverzweigten ($C_1$-$C_8$)-Alkylrest der einfach oder zweifach, insbesondere einfach substituiert ist mit

Hydroxy, $(C_1-C_{10})$-Alkoxy, $(C_1-C_8)$-Alkoxycarbonyl,$(C_3-C_8)$-Cycloalkoxycarbonyl,$(C_7-C_{12})$-Aralkoxycarbonyl, $(C_6-C_{10})$-Aryloxycarbonyl, Carbamoyl, N-($(C_1-C_8)$-Alkoxy-$(C_1-C_2)$alkyl)-carbamoyl, N-($(C_1-C_4)$-Hydroxyalkyl)carbamoyl oder ein O-acyliertes Derivat hiervon, $(C_1-C_{12})$-Alkylcarbonyloxy, Carboxyl, $(C_1-C_8)$-Alkoxycarbonyloxy, $(C_6-C_{12})$-Aryloxycarbonyloxy, $(C_7-C_{11})$-Aralkyloxycarbonyloxy, $(C_7-C_{11})$-Aralkylcarbonyloxy, $(C_6-C_{12})$-Arylcarbonyloxy, $(C_3-C_8)$-Cycloalkylcarbonyloxy, Carbamoyloxy, N-$(C_1-C_6)$-Alkylcarbamoyloxy, N,N-Di-$(C_1-C_8)$-alkylcarbamoyloxy, N-$(C_3-C_8)$-Cycloalkylcarbamoyloxy, $(C_1-C_8)$-Alkylcarbonyl, $(C_3-C_8)$-Cycloalkylcarbonyl, NR'R'', oder dem Acylrest einer N-derivatisierten Aminosäure, oder mit

einem substituierten $(C_6-C_{12})$Arylrest, der ein oder zwei Substituenten aus der Reihe Halogen, insbesondere Fluor, Chlor, Trifluormethyl, Hydroxy, $(C_1-C_4)$-Alkoxy, $(C_1-C_5)$-Alkyl, $(C_1-C_6)$-Alkylcarbonyloxy, $(C_1-C_6)$-Alkoxycarbonyl, $(C_1-C_4)$-Hydroxyalkyl aufweist, oder mit

einem substituierten $(C_6-C_{12})$-Aryloxyrest, $(C_7-C_{11})$-Aralkyloxyrest oder einem Heteroaryloxyrest, insbesondere einem Phenoxyrest oder einem Benzyloxyrest, der ein oder zwei Substituenten aus der Reihe Hydroxy, $(C_1-C_4)$-Alkoxy, $(C_1-C_5)$-Alkyl, $(C_1-C_6)$-Alkylcarbonyloxy, $(C_1-C_6)$-Alkoxycarbonyl, $(C_1-C_4)$-Hydroxyalkyl aufweist,

$R^6$ einen $(C_6-C_{12})$-Arylrest, der 1, 2 oder 3-fach substituiert ist mit Hydroxy, Halogen, insbesondere Chlor oder Brom, Cyano, Nitro, $(C_1-C_6)$-Alkyl, $(C_1-C_6)$-Alkoxy, Trifluormethyl, $(C_1-C_6)$-Hydroxyalkyl, $-O-[CH_2]_xCH_{(2f+1g)}F_g$. - $OCF_2Cl$, $OCF_2-CHFCl$, $(C_1-C_6)$-Alkylcarbonyl, $(C_1-C_6)$-Alkoxycarbonyl, Carbamoyl, N-$(C_1-C_8)$-Alkylcarbamoyl, N,N-Di-$(C_1-C_8)$-alkylcarbamoyl, $(C_1-C_6)$-Alkylcarbonyloxy, Phenyl, Benzyl, Phenoxy, Benzyloxy und/oder NR'R'',

$R^7$ unabhängig von $R^6$ Wasserstoff oder $(C_1-C_5)$Alkyl sein kann,

$R^5$ H oder eine Alkohol-Schutzgruppe sein kann, wobei $OR^5$ insbesondere einen substituierten oder unsubstituierten Methylether, einen substituierten oder unsubstituierten Ethylether, einen substituierten oder unsubstituierten Benzylether, einen Silylether, einen Ester, ein Carbonat, ein Carbamat oder ein Sulfonat bedeutet.

**3.** Verfahren nach den Ansprüchen 1 oder 2, in denen $R^5$ Wasserstoff, $(C_1-C_8)$-Alkyl, Benzyl, $(C_1-C_8)$-Alkylcarbonyl, Phenylcarbonyl und Carbamoyl sind.

**4.** Verfahren nach den Ansprüchen 1 bis 3, in denen n = 0 ist und $Y = CH_2OR^5$,

$R^1$ und $R^4$ sowie einer der Reste $R^2$ oder $R^3$, insbesondere $R^2$, Wasserstoff, $(C_1-C_5)$-Alkyl, Fluor, Chlor, $(C_1-C_5)$-Alkoxy,

und der andere Rest, $R^2$ oder $R^3$, insbesondere $R^3$ der allgemeinen Formel II entspricht, worin

X O,

$R^6$ verzweigtes oder unverzweigtes $(C_1-C_3)$-Alkyl, welches einfach substituiert ist mit Hydroxy, $(C_1-C_4)$-Alkoxy. $(C_1-C_9)$-Alkoxycarbonyl, $(C_3-C_6)$-Cycloalkoxycarbonyl, Benzyloxycarbonyl, $(C_1-C_4)$-Alkylcarbonyloxy und/oder ($(C_1-C_9)$-Alkoxycarbonyl)methyl)-carbamoyl, oder

$R^6$ $(C_6-C_{12})$Aryl, welches einfach oder zweifach substituiert ist mit Hydroxy, Halogen, $(C_1-C_6)$-Alkoxy, $(C_1-C_6)$-Alkyl, Trifluormethyl, $(C_1-C_6)$-Hydroxyalkyl, $-O-[CH_2]_xC_fH_{(2f+1-g)}F_g$ und/oder NR'R'', bedeutet und

$R^7$ Wasserstoff ist.

**5.** Verfahren nach den Ansprüchen 1 bis 4, in denen $R^6$ jeweils endständig substituiert ist.

**6.** Verfahren nach den Ansprüchen 1 bis 5, in denen $R^1$, $R^4$ sowie einer der beiden Reste $R^2$ oder $R^3$ Wasserstoff bedeuten.

**7.** Verfahren zur Herstellung von Verbindungen gemäß den Ansprüchen 1 bis 6, in denen $Y = -CH_2-OR^5$ bedeutet, dadurch gekennzeichnet, daß Pyridin-4- oder - 5-carbonsäuren der Formel VII, in denen Y' ein Substituent bedeutet, der sich in einen Rest $-CH_2-OR^5$ umwandeln läßt,

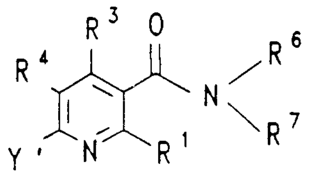

oder

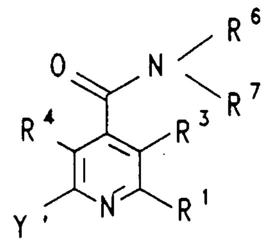

VII

a) sofern Y' = $CH_3$ und n = 0 bedeuten, zu Verbindungen der Formel VII halogeniert werden, in denen Y' = $CH_2W$ bedeutet, worin W für Halogen, insbesondere Chlor oder Brom, steht und diese Verbindungen ihrerseits mit Reagenzien der Formel $MOR^5$ zu Verbindungen der Formel I umgesetzt werden, worin M Wasserstoff, ein Alkali- oder ein Erdalkaliatom bedeutet, oder

b) sofern Y' = $CH_3$ und n = 1 bedeuten, mit $Ac_2O$/AcOH zu Verbindungen der Formel I umgesetzt werden, in denen Y = $-CH_2$-OAc bedeutet, oder

c) sofern Y' = $-CO_2H$, $-CO_2$Alkyl oder -CHO und n = 0 bedeuten, zu Verbindungen der Formel I, in denen Y = $CH_2OH$ bedeutet, reduziert werden.

**8.** Arzneimittel, enthaltend eine Verbindung der Formel I und einen verträglichen pharmazeutischen Träger.

**9.** Verwendung von Verbindungen der Formel I zur Beeinflussung des Stoffwechsels von Kollagen und kollagenähnlichen Stoffen.

**10.** Verwendung von Verbindungen der Formel I als Fibrosuppressiva.

**11.** Verfahren zur Herstellung von Arzneimitteln zur Beeinflussung des Stoffwechsels von Kollagen und kollagenähnlichen Stoffen, dadurch gekennzeichnet, daß das Arzneimittel eine Verbindung der Formel I enthält.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 11 5831
Seite 1

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| X,P | EP-A-0 480 258 (BAYER AG)<br>*Verbindungen 228-231, 462, 463, 473, 474, 481, 482*<br>--- | 1 | C07D213/81<br>C07D213/82<br>C07D213/89<br>A61K31/44 |
| X | CHEMICAL ABSTRACTS, vol. 82, no. 23, 9. Juni 1975, Columbus, Ohio, US;<br>abstract no. 156022n,<br>H.J. SATTLER ET AL. 'Nicethamide analogues. V. Hindered internal rotation in N,N-dimethylpyridinamides.'<br>Seite 580 ;<br>*CAS RN 55314-20-0*<br>* Zusammenfassung *<br>& CHEMISCHE BERICHTE<br>Bd. 108, Nr. 3, 1975, WEINHEIM, GER.<br>Seiten 730 - 734<br>--- | 1 | |
| X | EP-A-0 422 456 (BASF AKTIENGESELLSCHAFT)<br>* Beispiel 19 *<br>---<br>-/-- | 1-3 | |

| RECHERCHIERTE SACHGEBIETE (Int. Cl. 5) |
|---|
| C07D<br>A61K |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 02 DEZEMBER 1992 | P. BOSMA |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04E09)

Europäisches Patentamt

# EUROPÄISCHER TEILRECHERCHENBERICHT

Nummer der Anmeldung

EP 92 11 5831
Seite 2

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| X | CHEMICAL ABSTRACTS, vol. 95, no. 9, 1981, Columbus, Ohio, US; abstract no. 80670t, N.I. SHRAMM ET AL. 'Synthesis and biological activity of alkylamides of 2-arylamino-6-methylnicotinic acids.' Seite 758 ; *CAS RN 78593-68-7* * Zusammenfassung * & KHIMIKO FARMATSEVTICHESKII ZHURNAL Bd. 15, Nr. 4, 1981, MOSCOW Seiten 35 - 38 --- | 1 | |
| X | CHEMICAL ABSTRACTS, vol. 84, no. 23, 1976, Columbus, Ohio, US; abstract no. 164622q, I. NINOMIYA ET AL. 'Substituted pyridinecarboxamide derivatives.' Seite 452 ; *CAS RN 58446-37-0; 58446-40-5; 58446-41-6; 58446-44-9* * Zusammenfassung * & JP-A-75 121 283 (...) --- | 1-3,6 | RECHERCHIERTE SACHGEBIETE (Int. Cl. 5) |
| X | CHEMICAL ABSTRACTS, vol. 74, no. 25, 1971, Columbus, Ohio, US; abstract no. 141563q, I. MATSUMOTO ET AL. '4-Substituted pyridinemethanol carbamates.' Seite 570 ; *CAS RN 31804-64-5* * Zusammenfassung * & JP-A-7 040 286 (BANYU PHARMACEUTICAL CO., LTD.) --- | 1-3,6 | |
| X | CHEMICAL ABSTRACTS, vol. 74, no. 21, 1971, Columbus, Ohio, US; abstract no. 111926d, T. SHIMAMOTO ET AL. 'Nicotinic acid derivatives.' Seite 395 ; *CAS RN 24020-46-0* * Zusammenfassung * & JP-A-7 032 699 (...) --- | 1-3,6 | |

-/--

Europäisches Patentamt

# EUROPÄISCHER TEILRECHERCHENBERICHT

Nummer der Anmeldung

EP 92 11 5831
Seite 3

| EINSCHLÄGIGE DOKUMENTE | | | KLASSIFIKATION DER ANMELDUNG (Int. Cl. 5) |
|---|---|---|---|
| Kategorie | Kennzeichnung des Dokuments mit Angabe, soweit erforderlich der maßgeblichen Teile | Betrifft Anspruch | |
| A<br><br><br>D | EP-A-0 438 795 (HOECHST AKTIENGESELLSCHAFT)<br>* das ganze Dokument *<br>& DE-A-4 001 002<br>--- | 1-14 | |
| A,D | EP-A-0 353 668 (HOECHST AKTIENGESELLSCHAFT)<br>* das ganze Dokument *<br>----- | 1-14 | |
| | | | RECHERCHIERTE SACHGEBIETE (Int. Cl. 5) |

EPO FORM 1503 03.82 (P04E12)